# EUROPEAN PATENT APPLICATION

(11) **EP 3 650 539 A2**
(43) Date of publication of application: **13.05.2020**
(21) Application number: 18827529.1
(22) Date of filing: 09.07.2018
(51) Int. Cl.: C12N 9/16, A61K 47/68, A61K 38/00

(54) **NOVEL THERAPEUTIC ENZYME FUSION PROTEIN AND USE THEREOF**

(30) Priority: 07.07.2017 KR 20170086594
(71) Applicant: Hanmi Pharm. Co., Ltd., Hwaseong-si, Gyeonggi-do 18536 (KR)
(72) Inventor: HEO, Yong Ho, Hwaseong-si Gyeonggi-do 18469 (KR); KIM, Jin Young, Hwaseong-si Gyeonggi-do 18469 (KR); CHOI, In Young, Hwaseong-si Gyeonggi-do 18469 (KR); JUNG, Sung Youb, Hwaseong-si Gyeonggi-do 18469 (KR)
(74) Representative: Mewburn Ellis LLP
(86) International application number: PCT/KR2018/007754
(87) International publication number: WO 2019/009684

(57) **Abstract**

The present invention relates to a fusion protein between a therapeutic enzyme and an immunoglobulin Fc region, a method thereof, and a composition comprising the fusion protein.

## Description

### [Technical Field]

The present invention relates to a therapeutic enzyme fusion protein in which an immunoglobulin Fc region is fused to an enzyme for the purpose of increasing *in vivo* half-life of therapeutic enzymes, a method for its preparation, and a composition containing the same.

### [Background Art]

Lysosomes are cytoplasmatic organelles that function to degrade macromolecules such as proteins, polynucleotides, polysaccharides, and lipids. The internal environment of lysosomes is acidic, and hydrolase enzymes that promote the hydrolysis of biological macromolecules are contained therein. Lysosomes have also been found to have a certain role in the absorption of molecules through intracellular endocytosis.

Lysosomal storage disorders (hereinafter, LSDs) are inherited metabolic disorders characterized by loss of lysosomal functions. LSDs are caused by a deficiency of enzymes that degrade materials such as lipids, proteins, polysaccharides, *etc.,* and they usually occur with incidences of 1 in 100,000 and are inherited as autosomal recessive traits. LSDs appear when there is a deficiency or lack of specific degradative enzymes, and when these degradative enzymes are deficient, the resulting excess materials become accumulated without being degraded, eventually causing problems in cell functions. Like many other genetic disorders, LSDs are inherited from parents. Additionally, each of these diseases occurs by a mutation in any of the genes that are respectively involved in the translation of different enzymes. Enzymes that cause of these diseases usually have similar biochemical properties, and all of the LSDs are caused by abnormal accumulation of materials in the lysosomes. Currently, about 50 different types of LSDs are known (*e.g*., Niemann-Pick disease, Fabry's disease, Gaucher disease, Hunter syndrome, Maroteaux-Lamy syndrome, *etc.*)*.* A representative method for treating these LSDs may be enzyme-replacement therapy (ERT), and many related studies are currently underway (Frances M. Platt et al., J Cell Biol. 2012 Nov 26; 199 (5): 723 to 34).

Hunter syndrome, a representative of LSDs, is a disease caused by a deficiency of iduronate-2-sulfatase (IDS), in which glycosaminoglycan (GAG) is not degraded due to the deficiency of iduronate-2-sulfatase and accumulated in lysosomes. The symptoms of Hunter syndrome include a distinctive coarseness in facial features, large head, abdominal swelling due to hepatomegaly and splenomegaly, *etc.,* and it is also accompanied by hearing loss, heart valve disease, obstructive respiratory disease, sleep apnea, *etc.* Hunter syndrome is known to occur in 1 in 162,000 and is inherited as an X-linked recessive form associated with the X chromosome. Elaprase® (recombinant IDS, Shire Pharmaceuticals Group) is currently used as an enzyme-replacement therapy for the treatment of Hunter syndrome.

Generally, proteins such as therapeutic enzymes have low stability and are thus easily denatured and decomposed by proteases in the blood. Therefore, to maintain the blood concentration and potency of these proteins, frequent administration to patients is necessary. However, in the case of protein drugs administered to patients in the form of injections, frequent injections to maintain the blood concentration of active polypeptides may cause significant pain to the patient. To solve these problems, there has been a continuous effort to maximize pharmacological efficacy by increasing the blood stability of the therapeutic enzymes and maintaining their blood concentration at a high level for a longer period of time. Such long-acting formulations of therapeutic enzymes are required to increase the stability of therapeutic enzymes and to simultaneously maintain the potency of the drugs themselves at a sufficiently high level, as well as to cause no immune reaction in patients.

In particular, LSDs are fatal disorders caused by genetic defects in particular enzymes that can lead to death, and replacement therapy is essential for the treatment of the defective enzymes. Enzyme replacement therapy is a standard therapy in LSDs, and the therapy has an effect of alleviating the existing symptoms or delaying the progress of the disease by replacing the deficient enzyme. However, due to the requirement for continuous intravenous administration of a drug once every one or two weeks for 2 to 6 hours, the daily life of the patients and their family members may be restricted.

Since the half-lives of the recombinant enzymes used for the treatment of LSDs in humans are very short, in the range of 10 minutes to less than 3 hours, and the recombinant enzymes must be administered for the rest of one's life, it is thus inconvenient for patients. Accordingly, there is a high demand for the extension of the half-lives of the recombinant enzymes.

In order to stabilize proteins and prevent them from being removed in the kidney, fusion proteins using an immunoglobulin Fc region are currently being actively studied.

Immunoglobulins are major constituents of the blood, and there are five different types (*i.e.,* IgG, IgM, IgA, IgD, and IgE). The most frequently used type for fusion protein studies is IgG, and it is classified into four subtypes (IgG1∼IgG4). Fusion proteins prepared using an immunoglobulin Fc can increase their size and thereby prevent their being removed in the kidney and also bind to FcRn receptors, and thereby have the role of increasing blood half-life through endocytosis and recycling into cells.

However, an immunoglobulin Fc region has a disadvantage in that it can cause an unintended immune response, thereby having effector functions such as antibody-dependent cell-mediated cytotoxicity (ADCC) and complement-dependent cytotoxicity (CDC). These functions occur through the binding of an immunoglobulin Fc region to an Fc receptor or complement, or glycosylation of the Fc region. In addition, it is highly likely that instability of Fc itself may occur *in vivo.*

Therefore, there is a disadvantage in that the activity of the fused protein is not maintained while the duration of the desired fusion protein is simultaneously stably increased *in vivo.*

### [Disclosure]

### [Technical Problem]

An object of the present invention is to provide an enzyme fusion protein in which an immunoglobulin Fc region is fused to a therapeutic enzyme such that the therapeutic enzyme has increased *in vivo* duration compared to a therapeutic enzyme to which an immunoglobulin Fc region is not fused.

Another object of the present invention is to provide a pharmaceutical composition containing the therapeutic enzyme fusion protein.

Still another object of the present invention is to provide a polynucleotide encoding the therapeutic enzyme fusion protein, an expression vector containing the polynucleotide, and a transformant into which the expression vector is introduced.

Still another object of the present invention is to provide a method for preparing an enzyme fusion protein including culturing the transformant.

### [Technical Solution]

An aspect of the present invention provides an enzyme fusion protein in which a therapeutic enzyme and an immunoglobulin Fc region are fused.

In a specific embodiment, the present invention relates to an enzyme fusion protein, in which an immunoglobulin Fc region is fused to a therapeutic enzyme such that the therapeutic enzyme has increased *in vivo* duration compared to a therapeutic enzyme to which an immunoglobulin Fc region is not fused.

The following corresponds to a further embodiment of the present invention.

Specifically, as an enzyme fusion protein according to any one of the previous specific embodiments, the enzyme fusion protein is characterized in that the enzyme is selected from the group consisting of *beta*-glucosidase, *alpha*-galactosidase, *beta*-galactosidase, iduronidase, iduronate-2-sulfatase, galactose-6-sulfatase, acid *alpha*-glucosidase, acid ceramidase, acid sphingomyelinsase, galactocerebrosidsase, arylsulfatase A, B, *beta*-hexosaminidase A, B, heparin *N*-sulfatase, *alpha*-D-mannosidase, *beta*-glucuronidase, *N*-acetylgalactosamine-6 sulfatase, lysosomal acid lipase, *alpha*-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, and myeloperoxidase.

As an enzyme fusion protein according to any one of the previous specific embodiments, the enzyme fusion protein is characterized in that the therapeutic enzyme and the immunoglobulin Fc region in the enzyme fusion protein are fused by a peptide linker.

As an enzyme fusion protein according to any one of the previous specific embodiments, the enzyme fusion protein is characterized in that an immunoglobulin Fc region molecule and a dimeric therapeutic enzyme are fused.

As an enzyme fusion protein according to any one of the previous specific embodiments, the enzyme fusion protein is characterized in that the immunoglobulin Fc region has a variation selected from the group consisting of substitution, addition, deletion, modification, and a combination thereof in at least one amino acid of a native immunoglobulin Fc region.

As an enzyme fusion protein according to any one of the previous specific embodiments, the enzyme fusion protein is characterized in that, in the immunoglobulin Fc region having the amino sequence of SEQ ID NO: 8, the 2^{nd} amino acid is substituted with proline; the 71^{st} amino acid is substituted with glutamine; or the 2^{nd} amino acid is substituted with proline and the 71^{st} amino acid is substituted with glutamine.

As an enzyme fusion protein according to any one of the previous specific embodiments, the enzyme fusion protein is characterized in that no chain exchange occurs in the immunoglobulin Fc region.

As an enzyme fusion protein according to any one of the previous specific embodiments, the enzyme fusion protein is characterized in that it has increased stability and reduced binding affinity for lysosome receptors, thus having a high degree of tissue distribution compared to a therapeutic enzyme to which an immunoglobulin Fc region is not fused.

As an enzyme fusion protein according to any one of the previous specific embodiments, the enzyme fusion protein is characterized in that the immunoglobulin Fc region is selected from the group consisting of (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination of one or two or more domains among a CH1 domain, a CH2 domain, a CH3 domain, and a CH4 domain and an immunoglobulin hinge region or a part of the hinge region; and (f) a dimer between each domain of the heavy chain constant region and the light chain constant region.

As an enzyme fusion protein according to any one of the previous specific embodiments, the enzyme fusion protein is characterized in that the immunoglobulin Fc region has at least one characteristic selected from the group consisting of (a) removal of a region capable of forming a disulfide bond, (b) removal of a certain amino acid residue at the N-terminus of a native Fc, (c) addition of a methionine residue at the N-terminus of a native Fc, (d) removal of a complement-binding site, or (e) deletion of an antibody-dependent cell-mediated cytotoxicity (ADCC) site.

As an enzyme fusion protein according to any one of the previous specific embodiments, the enzyme fusion protein is characterized in that the immunoglobulin Fc region is aglycosylated.

As an enzyme fusion protein according to any one of the previous specific embodiments, the enzyme fusion protein is characterized in that the immunoglobulin Fc region is an Fc fragment derived from IgG, IgA, IgD, IgE, or IgM.

As an enzyme fusion protein according to any one of the previous specific embodiments, the enzyme fusion protein is characterized in that the immunoglobulin Fc region is a hybrid of domains having different origins derived from immunoglobulins selected from the group consisting of IgG, IgA, IgD, IgE, and IgM.

As an enzyme fusion protein according to any one of the previous specific embodiments, the enzyme fusion protein is characterized in that the immunoglobulin Fc region is an IgG4 Fc region.

As an enzyme fusion protein according to any one of the previous specific embodiments, the enzyme fusion protein is characterized in that the hinge region of the IgG4 Fc region is substituted with an IgG1 hinge region.

Another aspect of the present invention provides a pharmaceutical composition for preventing or treating lysosomal storage disorder (LSD).

In a specific embodiment, the present invention relates to a pharmaceutical composition for preventing or treating LSD containing the enzyme fusion protein.

As a composition according to any one of the previous specific embodiments, the composition is characterized in that LSD is selected from the group consisting of mucopolysaccharidosis (MPS), glycogen storage disease, sphingolipidosis, Niemann-Pick disease, Fabry's disease, Gaucher disease, Hunter syndrome, and Maroteaux-Lamy syndrome.

As a composition according the previous specific embodiments, the composition is characterized in that the enzyme is iduronate-2-sulfatase (IDS) or arylsulfatase B (ARSB).

As a composition according to any one of the previous specific embodiments, the composition is characterized in that it reduces the binding affinity of a therapeutic enzyme for lysosome receptors.

Still another aspect of the present invention provides a polynucleotide encoding the enzyme fusion protein.

Still another aspect of the present invention provides an expression vector containing the polynucleotide.

Still another aspect of the present invention provides a transformant into which the expression vector is introduced.

Still another aspect of the present invention provides a method for preparing an enzyme fusion protein.

In a specific embodiment, the present invention relates to a method for preparing an enzyme fusion protein, which includes culturing the transformant to obtain a culture; and recovering an enzyme fusion protein from the culture.

### [Advantageous Effects of the Invention]

The present invention relates to a long-acting therapeutic enzyme fusion protein, and specifically, to an enzyme fusion protein in which an immunoglobulin Fc region is fused to a therapeutic enzyme such that the therapeutic enzyme has increased stability and the mechanism of enzyme removal by the kidney is reduced. The enzyme fusion protein of the present invention can be effectively used by patients due to the increased duration of time.

### [Brief Description of Drawings]

FIG. 1 shows a graph confirming the expression of an IDS-Fc fusion protein.
FIG. 2 shows a graph confirming the expression of an ARSB-Fc fusion protein.
FIG. 3 shows a graph confirming the results of pharmacokinetic experiments of IDS-Fc fusion protein of the present invention.
FIG. 4 shows a graph confirming the results of pharmacokinetic experiments of ARSB-Fc fusion protein of the present invention.
FIG. 5 shows a graph confirming the *in vitro* enzyme activity of IDS-Fc fusion protein of the present invention.
FIG. 6 shows a graph confirming the *in vitro* enzyme activity of ARSB-Fc fusion protein of the present invention.
FIG. 7 shows a graph illustrating the measurement results of glycosaminoglycan (GAG) levels in urine after intravenous or subcutaneous injection of IDS-Fc fusion protein of the present invention, into an IDS-knockout mouse.
FIG. 8 shows a graph illustrating the measurement results of glycosaminoglycan (GAG) levels in tissue after intravenous or subcutaneous injection of IDS-Fc fusion protein of the present invention, into an IDS-knockout mouse.
FIG. 9 shows a graph confirming the degree of tissue distribution of ARSB-Fc fusion protein of the present invention.

### [Detailed Description of the Invention]

Hereinbelow, exemplary embodiments of the present invention will be described in detail. Meanwhile, each of the explanations and exemplary embodiments disclosed herein can be applied to other explanations and exemplary embodiments. That is, all of the combinations of various factors disclosed herein belong to the scope of the present invention. Furthermore, the scope of the present invention should not be limited by the specific disclosure provided hereinbelow.

Additionally, those skilled in the art will be able to recognize or confirm, based on routine experimentation, many equivalents to the specific embodiments of the present invention described in this application, and such equivalents are intended to be included in the present invention.

Throughout the entire specification, not only the conventional one-letter or three-letter codes for naturally occurring amino acids, but also those three-letter codes generally allowed for other amino acids are used, such as α-aminoisobutyric acid (Aib), Sar(*N*-methylglycine), α-methyl-glutamic acid, *etc.* Additionally, the amino acids mentioned in abbreviations herein are described according to the IUPAC-IUB rules as follows:

| | | | |
|---|---|---|---|
| Alanine | A; | Arginine | R; |
| Asparagine | N; | Aspartic acid | D; |
| Cysteine | C; | Glutamic acid | E; |
| Glutamine | Q; | Glycine | G; |
| Histidine | H; | Isoleucine | I; |
| | | | |
| Leucine | L; | Lysine | K; |
| Methionine | M; | Phenylalanine | F; |
| Proline | P; | Serine | S; |
| Threonine | T; | Tryptophan | W; |
| Tyrosine | Y; and | Valine | V. |

An aspect of the present invention provides an enzyme fusion protein in which an immunoglobulin Fc region is fused to a therapeutic enzyme such that the therapeutic enzyme has increased *in vivo* duration compared to a therapeutic enzyme to which an immunoglobulin Fc region is not fused.

In the present invention, the enzyme fusion protein may be one in which an immunoglobulin Fc region is fused to a therapeutic enzyme such that the therapeutic enzyme can maintain its activity while its binding affinity for lysosome receptors is reduced, compared to a therapeutic enzyme to which an immunoglobulin Fc region is not fused, thereby increasing its blood half-life.

The present inventors have prepared a fusion protein with an immunoglobulin Fc region to increase the blood half-life of therapeutic enzymes. In particular, as the Fc region, an IgG4 Fc analog was used in which a potential glycosylation sequence is substituted to inhibit glycosylation and additionally a hinge sequence of IgG4 Fc is substituted to inhibit chain exchange. As a result, the present inventors have confirmed that the blood half-life of the therapeutic enzyme fusion protein fused to an immunoglobulin Fc region has a significantly increased blood half-life and is able to maintain an activity similar to those of known enzymes, thereby providing a novel form of fusion protein structure in which a therapeutic enzyme and an immunoglobulin Fc region are fused.

The therapeutic enzyme to be included in the enzyme fusion protein of the present invention may include any therapeutic enzyme that can have an advantage of extended *in vivo* duration over a type of therapeutic enzyme to which an immunoglobulin Fc region is not fused, but the therapeutic enzyme is not particularly limited thereto. In an exemplary embodiment of the present invention, the enzyme fusion protein is a fusion protein of a therapeutic enzyme.

Additionally, the enzyme fusion protein of the present invention may be used as a drug for enzymatic replacement therapy (ERT). The enzymatic replacement therapy can prevent or treat a disease through recovery of the function of a deteriorated enzyme by supplementing the defective or deficient enzyme that causes the disease.

In a specific embodiment, the therapeutic enzyme may be a therapeutic enzyme selected from the group consisting of *beta*-glucosidase, *alpha*-galactosidase, *beta*-galactosidase, iduronidase, iduronate-2-sulfatase, galactose-6-sulfatase, acid *alpha*-glucosidase, acid ceramidase, acid sphingomyelinsase, galactocerebrosidsase, arylsulfatase A, B, *beta*-hexosaminidase A, B, heparin *N*-sulfatase, *alpha*-*D*-mannosidase, *beta*-glucuronidase, *N*-acetylgalactosamine-6 sulfatase, lysosomal acid lipase, *alpha*-*N*-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, and myeloperoxidase, but any therapeutic enzyme having a therapeutic effect on diseases may be included in the present invention regardless of its origin or type.

In the present invention, the term "enzyme fusion protein" may be used interchangeably with "long-acting enzyme fusion protein".

As used herein, the term "therapeutic enzyme" refers to an enzyme for treating diseases that occur due to lack, deficiency, malfunction, *etc.,* and the enzyme can treat a subject with the diseases by enzyme replacement therapy, administration, *etc.* Specifically, the enzyme may be an enzyme for treating LSDs that may occur due to the lack, deficiency, *etc.* of lysosomal enzyme, but the enzyme is not limited thereto.

Specifically, the therapeutic enzyme of the present invention may be arylsulfatase B (ARSB) or iduronate-2-sulfatase, but the therapeutic enzyme is not limited thereto as long as it is an enzyme that exhibits a therapeutic effect on target diseases.

As used herein, the term "arylsulfatase B (ARSB)" refers to an arylsulfatase which is present in the lysosomes of the liver, pancreas, and kidneys, and the enzyme has the role of hydrolyzing sulfates by decomposing glycosaminoglycan. The arylsulfatase B is known to be associated with mucopolysaccharidosis VI (Maroteaux-Lamy syndrome). In the present invention, the term arylsulfatase B may be used interchangeably with galsulfase. Specifically, the arylsulfatase B may include the amino acid sequence of SEQ ID NO: 4 which can be encoded by the polynucleotide sequence of SEQ ID NO: 3, but the arylsulfatase B is not limited thereto.

As used herein, the term "iduronate-2-sulfatase" is a sulfatase associated with Hunter syndrome (MPS-II) and it is an enzyme essential for lysosomal degradation of heparin sulfate and dermatan sulfate. In the present invention, the term iduronate-2-sulfatase may be used interchangeably with idursulfase. The idursulfase may be idursulfase alpha or idursulfase beta, but the idursulfase is not limited thereto. Specifically, the iduronate-2-sulfatase may include the amino acid sequence of SEQ ID NO: 2 which can be encoded by the polynucleotide sequence of SEQ ID NO: 1, but the iduronate-2-sulfatase is not limited thereto.

The therapeutic enzyme may be prepared or manufactured by a method known in the art, and specifically, the enzyme may be purified from the culture after culturing animal cells into which an animal expression vector is inserted, or may be used after purchasing commercially available enzymes, but the enzyme is not limited thereto.

The enzyme fusion proteins of the present invention may be in a form where one or two enzymes are bound one molecule of an Fc region having two immunoglobulin chains in a dimeric form, but the enzyme fusion proteins are not limited thereto. Specifically, the enzyme fusion proteins of the present invention may be in a form where a monomeric Fc region and an enzyme are fused and expressed, and then, two monomeric Fc regions form one molecule of a dimeric Fc region through a disulfide bond, and each of the two enzymes is linked to each of the two Fc regions, but the enzyme fusion proteins are not limited thereto. The enzymes may be linked to each other through a covalent or non-covalent bond, or may be independent from each other, but the enzyme fusion proteins are not limited thereto.

Specifically, in an embodiment of the present invention, it was confirmed that a long-acting enzyme fusion protein, in which a dimer of iduronate-2-sulfatase or arylsulfatase B and one molecule of Fc region are fused, exhibits a higher *in vitro* enzyme activity compared to an enzyme to which an Fc region is not fused, and it was confirmed that this is due to the structural characteristics of enzyme fusion proteins which include a dimer of therapeutic enzymes (Example 5).

Additionally, in another aspect, the enzyme fusion protein of the present invention may be one in which an immunoglobulin Fc region is fused to a therapeutic enzyme through a peptide linker.

The peptide linker may include one or more amino acids, for example, 1 to 1,000 amino acids, but the peptide linker is not particularly limited thereto. In the present invention, any known peptide linker (*e.g.,* including [GS]ₓ linker, [GGGS]ₓ linker, and [GGGGS]ₓ linker, *etc.,* in which x is a natural number of 1 or greater (*e.g.,* 1, 2, 3, 4, 5, or greater), and more specifically, the amino acid sequence of SEQ ID NO: 6, but the peptide linkers are not limited thereto.

For the purpose of the present invention, the position at which a peptide linker is fused to a therapeutic enzyme and an immunoglobulin Fc is not limited as long as the peptide linker can link the therapeutic enzyme and the immunoglobulin Fc while maintaining the activity of the therapeutic enzyme, specifically, both ends of the therapeutic enzyme and the immunoglobulin Fc region, and more specifically, the C-terminus of the therapeutic enzyme and the N-terminus of the immunoglobulin Fc region, but the position is not limited thereto.

As used herein, the terms "N-terminus" and "C-terminus" refer to an amino end and a carboxyl end of a protein, respectively. For example, "N-terminus" or "C-terminus" may include not only the most terminal amino acid residue of the N-terminus or C-terminus, but also the amino acid residues adjacent to the amino acid residue of the N-terminus or C-terminus, and specifically, the 1^{st} amino acid residue to the 20^{th} amino acid residue from the terminus itself, but the N-terminus or C-terminus is not particularly limited thereto.

In an embodiment of the present invention, fusion proteins (SEQ ID NO: 23 or 25) in which the N-terminus of IgG4 is fused to the C-terminus of a therapeutic enzyme were prepared using the therapeutic enzyme and a linker (SEQ ID NO: 6)-IgG4 by overlapping PCR, and the expression of the fusion proteins were confirmed (Examples 1 to 3).

The therapeutic enzyme included in the enzyme fusion protein of the present invention may be of a naturally occurring type, and a fragment consisting of a part of the therapeutic enzyme, or an analog of the therapeutic enzyme in which a variation selected from the group consisting of substitution, addition, deletion, and modification of some amino acids, and a combination thereof has occurred, may be included in the present invention without limitation, as long as it has an activity equivalent to that of a naturally occurring type of therapeutic enzyme.

Additionally, the analog of the therapeutic enzyme includes all of those where one or more amino acids are added to the amino and/or carboxy terminus of the naturally occurring type of therapeutic enzyme.

For the substitution or addition of amino acids, not only the 20 amino acids commonly found in human proteins, but also atypical or non-naturally occurring amino acids may be used. Commercial sources of the atypical amino acids may include Sigma-Aldrich, ChemPep Inc., Genzyme Pharmaceuticals, *etc.* The peptides including these amino acids and atypical peptide sequences may be synthesized and purchased from commercial suppliers, *e.g.,* American Peptide Company, Bachem (USA), or Anygen (Korea), but the commercial sources are not limited thereto.

As used herein, the term "fragment" refers to a form where one or more amino acids in the amino or carboxy terminus of a native therapeutic enzyme or an analog of a native therapeutic enzyme are removed. The native therapeutic enzyme or an analog thereof belongs to the scope of the present invention regardless of the size of the fragment or the kind of amino acids as long as they have an activity of a therapeutic enzyme.

The therapeutic enzyme analogs may include the biosimilars and biobetters of the corresponding therapeutic enzymes. For example, with respect to biosimilars, considering the difference in a host for its expression compared to a known therapeutic enzyme, the difference in glycosylation feature and the degree thereof, and the difference in the degree of substitution in a particular amino acid residue of the corresponding enzyme in light of the standard sequence where the degree of substitution is not 100% substitution, they belong to the biosimilar enzymes to be used as the enzyme fusion protein of the present invention. The therapeutic enzymes may be produced by a known method in the art, specifically by genetic recombination in animal cells, *E*. *coli,* yeast, insect cells, plant cells, live animals, *etc.,* and the preparation method is not limited thereto, and commercially available enzymes may be purchased and used, but the enzymes are not limited thereto.

Additionally, the therapeutic enzymes may include an amino acid sequence which has a homology of at least 80%, more specifically 90%, and even more specifically 91%, 92%, 93%, 94%, 95%, 96%, 97%, 98%, or 99% or higher to that of the above enzymes or analogs thereof, and the therapeutic enzymes may be obtained from microorganisms by recombinant technology or those which are commercially available, but the therapeutic enzymes are not limited thereto.

As used herein, the term "homology" represents the degree of similarity to the wild-type amino acid sequence or a wild-type nucleotide sequence, and the homology comparison can be performed by the naked eye or using a comparison program that can easily be purchased. The homologies between two or more sequences can be calculated as a percentage (%) using a commercial computer program. The homology (%) may be calculated for the neighboring sequences.

The information on the sequences of the therapeutic enzymes or analogs thereof and the nucleotide sequences encoding the same can be obtained from a known database (*e.g.,* NCBI, *etc.*)*.*

As used herein, the term "immunoglobulin Fc region" refers to a region of an immunoglobulin molecule including the heavy chain constant region 2 (CH2) and/or the heavy chain constant region 3 (CH3), excluding the variable regions of the heavy and light chains. For the purpose of the present invention, such an immunoglobulin Fc region may include a modified hinge region at the heavy chain constant region, but is not limited thereto.

Such an immunoglobulin Fc region may include a hinge region in the heavy chain constant region, but is not limited thereto. Additionally, the immunoglobulin Fc region of the present invention may be an extended Fc region including a part or the entirety of the heavy chain constant region 1 (CH1) and/or the light constant region 1 (CL1), excluding the variable regions of the heavy and light chains of an immunoglobulin, as long as the immunoglobulin Fc region has an effect the same as or equivalent to that of its native type. Additionally, the immunoglobulin Fc region of the present invention may be a region in which a part of a significantly long amino acid sequence corresponding to CH2 and/or CH3 is removed.

In another aspect, the present invention provides an immunoglobulin Fc region which may be selected from the group consisting of 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain, and a CH4 domain and an immunoglobulin hinge region (or a part of the hinge region); and 6) a dimer between each domain of the heavy chain constant region and the light chain constant region, but the immunoglobulin Fc region is not limited thereto.

As used herein, the term "chain exchange" refers to a problem in that when an IgG4 Fc is used as a carrier of a fusion protein, the IgG4 Fc forms a hybrid with an IgG4 present *in vivo* or is present as a monomer and alters the original structure to have a structure with a low therapeutic activity, and it was previously reported that there is significant difficulty when a fusion protein, in which a protein is fused, is used for therapeutic purposes (van der Neut Kolfschoten, et al., Science, 317:1554 to 1557. 2007).

In the present invention, the present inventors have made efforts to solve the above problem by substituting the sequence of a hinge region in an immunoglobulin Fc region. Specifically, the immunoglobulin Fc region of the present invention may be one in which a potent glycosylation sequence is substituted for the regulation of glycosylation or the sequence involved in chain exchange is substituted, or may correspond to both cases.

In a specific embodiment, the immunoglobulin Fc region of the present invention may be one in which the 2^{nd} amino acid and/or the 71^{st} amino acid of the immunoglobulin Fc region of SEQ ID NO: 8 is substituted with a different amino acid for the prevention of chain exchange and N-glycosylation. More specifically, the immunoglobulin Fc region of the present invention may be 1) one in which the 2^{nd} amino acid (*i.e.,* serine) is substituted with proline, 2) one in which the 71^{st} amino acid (*i.e.,* asparagine) is substituted with glutamine, but the immunoglobulin Fc region is not limited thereto. In addition to the variations described above, the immunoglobulin Fc region may include an appropriate variation as a drug carrier for increasing stability of a therapeutic enzyme.

Specifically, the immunoglobulin Fc region may be one in which a hinge region of an immunoglobulin IgG4 Fc is substituted with an IgG1 hinge region, but the immunoglobulin Fc region is not limited thereto.

In an embodiment of the present invention, the 2^{nd} amino acid of the immunoglobulin Fc region of SEQ ID NO: 8 is substituted with proline and the 71^{st} amino acid of the immunoglobulin Fc region of SEQ ID NO: 8 is substituted with glutamine, and thereby chain exchange and N-glycosylation were reduced. The sequence of the prepared immunoglobulin Fc has the amino acid sequence of SEQ ID NO: 9 (Example 1).

In an embodiment, the hinge region may be one in which a part of the hinge sequence having the following amino acid sequence is deleted or modified.

### Glu-Ser-Lys-Tyr-Gly-Pro-Pro-Cys-Pro-Ser-Cys-Pro (SEQ ID NO: 26)

Specifically, the hinge region may be one having a variation where a part of the hinge region is deleted to include only one cysteine (Cys) residue; or may be one where a serine (Ser) residue involved in chain exchange is substituted with a proline (Pro) residue, and more specifically, one where the 2^{nd} serine of the hinge sequence is substituted with a proline residue, but the hinge region is not limited thereto.

In the present invention, an immunoglobulin Fc region can increase the stability of a fused therapeutic enzyme while preventing the chain exchange and formation of monomers in an Fc region by including a hinge region in its native form or a modified hinge region.

Additionally, in another specific embodiment, the immunoglobulin Fc region of the present invention not only includes native amino acid sequences but also sequence analogs thereof. An amino acid analog means that a variation selected from the group consisting of substitution, addition, deletion, modification, and a combination thereof has occurred in at least one amino acid residue of a native amino acid sequence.

For example, amino acid residues at positions 214 to 238, 297 to 299, 318 to 322, or 327 to 331 in IgG Fc, which are known to be important for linkage, may be used as the sites suitable for variation. Additionally, various types of analogs are possible, for example, one where the site capable of forming a disulfide bond is removed, one where several N-terminal amino acids from native Fc are removed, one where a methionine residue is added to the N-terminus of native Fc, *etc.* Additionally, complement binding sites (*e.g.,* C1q binding sites) or antibody-dependent cell-mediated cytotoxicity (ADCC) sites may be removed to remove the effector function. The techniques for preparing the sequence analogs of an immunoglobulin Fc region are disclosed in International Publication Nos. WO 97/34631, WO 96/32478, *etc.*

Amino acid substitutions in a protein or peptide molecule that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, *etc.*

Additionally, the Fc analogs described above may be those which exhibit the same biological activity as that of the Fc region of the present invention, and have increased structural stability of the Fc region against heat, pH, *etc.*

Additionally, such an Fc region may be obtained from a native type isolated from humans or animals such as cows, goats, pigs, mice, rabbits, hamsters, rats, guinea pigs, *etc.,* or may be their recombinants or analogs obtained from transformed animal cells or microorganisms. Herein, they may be obtained from a native Fc by isolating whole immunoglobulins from human or animal organisms and treating them with a protease. Papain digests the native Fc region into Fab and Fc regions, and pepsin treatment results in the production of pF'c and F(ab)₂ fragments. These fragments may be subjected to size exclusion chromatography to isolate Fc or pF'c. In a more specific embodiment, the Fc region may be a recombinant immunoglobulin Fc region obtained from a microorganism, which is a human-derived Fc region.

Additionally, the immunoglobulin Fc region may be in the form of native glycan, increased or decreased glycans compared to its native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc region where the glycans are removed from the Fc region shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc region in a deglycosylated or aglycosylated immunoglobulin Fc region may be a more suitable form to meet the original object of the present invention as a drug carrier.

As used herein, the term "deglycosylation" refers to a removal of sugar moieties from an Fc region by an enzyme, and the term "aglycosylation" refers to an unglycosylated Fc region produced in prokaryotes, more specifically, *E. coli.*

Meanwhile, the immunoglobulin Fc region may be derived from humans or animals including cows, goats, pigs, mice, rabbits, hamsters, rats, and guinea pigs, and more specifically, it may be derived from humans.

Additionally, the immunoglobulin Fc region may be an Fc region derived from IgG, IgA, IgD, IgE, IgM, or a combination or hybrid thereof. In a more specific embodiment, it may be derived from IgG or IgM, which are among the most abundant proteins in human blood, and in an even more specific embodiment, it may be derived from IgG, which is known to enhance the half-lives of ligand-binding proteins. In a more specific embodiment, the immunoglobulin Fc region may be an IgG4 Fc region, in an even more specific embodiment, it may be an aglycosylated Fc region derived from a human IgG4, and in a most specific embodiment, the immunoglobulin Fc region may be an IgG4 Fc region which includes a variation where the 2^{nd} amino acid having the amino acid sequence of SEQ ID NO: 8 of the immunoglobulin Fc region is substituted with proline and/or the 71^{st} amino acid is substituted with glutamine; or the amino acid sequence of the immunoglobulin Fc region is SEQ ID NO: 9 and the polynucleotide encoding the amino acid sequence is SEQ ID NO: 7, but the immunoglobulin Fc region is not limited thereto.

As used herein, the term "combination" means that polypeptides encoding single-chain immunoglobulin Fc regions of the same origin are linked to a single-chain polypeptide of a different origin to form a dimer or multimer. That is, a dimer or multimer may be prepared from two or more fragments selected from the group consisting of Fc fragments of IgG Fc, IgA Fc, IgM Fc, IgD Fc, and IgE Fc.

Additionally, the proteins of the present invention may be those where the N-terminus and/or C-terminus of the proteins are not modified, but, for protecting and increasing stability of the therapeutic enzymes from protein cleavage enzymes *in vivo,* those proteins where the N-terminus and/or C-terminus of the therapeutic enzymes are chemically modified or protected by organic group, or the amino terminus of the therapeutic enzymes is modified by the addition of an amino acid, *etc.* are also included in the scope of the proteins according to the present invention. When the C-terminus of the therapeutic enzymes is not modified, the termini of the proteins according to the present invention may have a carboxyl terminus, but the proteins of the present invention are not particularly limited thereto.

In particular, since the N-terminus and C-terminus of chemically synthesized proteins have charges, the N-terminus may be acetylated and/or C-terminus may be amidated so as to remove these charges, but the methods are not particularly limited thereto.

Unless specified otherwise in the present specification, the technologies with regard to "enzyme" or "fusion protein" according to the present invention described in the detailed description or claims of the present invention will be applied not only to the subject enzyme or fusion protein, but also to the scope which includes all of the salts of the subject enzyme or fusion protein (*e.g.,* a pharmaceutically acceptable salt of the fusion protein), or a solvate thereof. Accordingly, although it is simply described as "enzyme" or "fusion protein" in the specification, the subject description will be likewise applied to the specific salt, the specific solvate, and the specific solvate of the specific salt. Such salt forms may be in a form, for example, using any pharmaceutically acceptable salt, but the kind of the salt is not particularly limited. Those salt forms, for example, may be those which are safe and effective to mammals, but the salt forms are not particularly limited thereto.

As used herein, the term "pharmaceutically acceptable" refers to a material which can be effectively used for the intended use without causing excessive toxicity, stimulation, or allergic reactions, *etc.* within the range of medico-pharmaceutical decision.

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.;* alkali earth metals such as magnesium; ammonium, *etc.*

Additionally, as used herein, the term "solvate" refers to a complex formed between the enzyme, fusion protein according to the present invention or a salt thereof and a solvent molecule.

The enzyme fusion protein of the present invention may be prepared by a method known in the art.

In an embodiment of the present invention, a recombinant vector was prepared where each of iduronate-2-sulfatase (IDS) and arylsulfatase B (ARSB) (*i.e.,* therapeutic enzymes) can be expressed in a form fused to a peptide linker-immunoglobulin Fc, and these therapeutic enzymes were prepared by expressing them in a CHO cell line (Examples 1 to 3).

However, the enzyme fusion protein of the present invention may be prepared by methods other than those described in the above embodiments. The enzyme fusion protein may include the amino acid sequence of SEQ ID NO: 23 or 25, but the amino acid sequences are not limited thereto.

The enzyme fusion protein according to the present invention can increase the half-life of a therapeutic enzyme that exhibits a therapeutic effect on LSDs while maintaining the activity of the therapeutic enzyme, by fusing the therapeutic enzyme to an immunoglobulin Fc region. In particular, a therapeutic enzyme fused to a modified immunoglobulin Fc region has reduced chain exchange and glycosylation, and thus can have a lower binding affinity for lysosome receptors compared to a therapeutic enzyme to which an Fc is not fused, and can thereby have high duration, confirming that such a therapeutic enzyme is effective for the treatment of LSDs.

In an embodiment of the present invention, it was confirmed that the enzyme fusion protein according to the present invention can maintain *in vitro* enzyme activity (Example 5) while having a significantly excellent half-life (T_{1/2}), maximum drug concentration in blood (Cₘₐₓ), and *in vivo* availability (AUC) (Example 4) compared to a naturally occurring enzyme to which an Fc region is not fused, and from these results, it was confirmed that the drug can be used even at low doses compared to conventional drugs (Example 6).

Still another aspect of the present invention provides a pharmaceutical composition for preventing or treating lysosomal storage disorders (LSDs) containing an enzyme fusion protein, which is prepared according to the method for preparing an enzyme fusion protein or enzyme fusion protein.

The composition according to the present invention is characterized in that the *in vivo* duration and stability of a therapeutic enzyme are increased.

In a specific embodiment, the enzyme fusion protein of a pharmaceutical composition of the present invention may be those where iduronate-2-sulfatase (IDS) or arylsulfatase B (ARSB) is fused to an immunoglobulin Fc region, but the enzyme fusion protein is not limited thereto.

As used herein, the term "lysosome", being one of the organelles present in the cytoplasm, contains many hydrolases and thus decomposes unwanted materials in the body such as macromolecules, bacteria, *etc.,* and helps the decomposed products to be utilized in other parts of cells. The functions of a lysosome can be performed by many enzymes. When a particular enzyme loses its function due to a mutation, deficiency, *etc.,* it causes the loss of the decomposing function of the lysosome and results in the accumulation of macromolecules, *etc.,* which must be decomposed, in the cell and induce cell damage, *etc.* thereby causing a disease.

As used herein, the term "lysosomal storage disease (LSD)" refers to a rare genetic disease due to the loss of lysosomal functions described above, and enzymatic replacement therapy using a defective enzyme is essential. According to the deficient enzyme, LSD may include mucopolysaccharidosis (MPS), glycogen storage disease, sphingolipidosis, Niemann-Pick disease, Fabry's disease, Gaucher disease, Hunter syndrome, Maroteaux-Lamy syndrome, *etc.*

Hereinafter, LSD will be described in detail according to its classification.

As used herein, the term "Maroteaux-Lamy syndrome", which belongs to type VI mucopolysaccharidosis (MPS) diseases, is an autosomal recessive genetic disease that occurs due to the deficiency of arylsulfatase B (*N*-acetylgalactosamine-4-sulfatase) necessary for the breakdown of glycosaminoglycan. Maroteaux-Lamy syndrome is a disease that occurs by the deposition of dermatan sulfate which was not decomposed due to the deficiency of the enzyme in the bones, cardiac valves, spleen, liver, cornea, *etc.*

As used herein, the term "arylsulfatase B (ARSB)" refers to an arylsulfatase which is present in the lysosomes of the liver, pancreas, and kidneys, and the enzyme has the role of hydrolyzing sulfates by decomposing glycosaminoglycan. The arylsulfatase B is known to be associated with mucopolysaccharidosis VI (Maroteaux-Lamy syndrome). In the present invention, the term arylsulfatase B may be used interchangeably with galsulfase.

As used herein, the term "Hunter syndrome (Hunter disease)" is a X-linked recessive genetic disease that occurs due to the deficiency of iduronate-2-sulfatase (IDS), and it is known that heparan sulfate and dermatan sulfate are accumulated due to the deficiency of the enzyme. Symptoms of Hunter syndrome include deterioration in functions, progressive hearing loss, retinitis pigmentosa, papilledema, hydrocephalus, *etc.* In the present invention, the term "mucopolysaccharidosis II" may be used interchangeably with "Hunter syndrome".

As used herein, the term "iduronate-2-sulfatase", which is a sulfatase enzyme related to Hunter syndrome (MPS-II), is an enzyme necessary for lysosomal degradation of heparin sulfate and dermatan sulfate. In the present invention, the term "iduronate-2-sulfatase" may be used interchangeably with "idursulfase". The idursulfase may be, for example, idursulfase alpha or idursulfase beta, but the idursulfase is not limited thereto.

The therapeutic enzyme may be prepared or manufactured by a method known in the art, and specifically, the enzyme may be purified from the culture after culturing animal cells into which an animal expression vector is inserted, or may be used after purchasing commercially available enzymes, but the enzyme is not limited thereto.

The enzyme fusion protein contained in the composition of the present invention can increase the half-life of a therapeutic enzyme that exhibits a therapeutic effect on LSDs while maintaining the activity of the therapeutic enzyme by fusing the therapeutic enzyme to an immunoglobulin Fc region. In particular, a therapeutic enzyme fused to a modified immunoglobulin Fc region has reduced chain exchange and glycosylation, and thus can have a lower binding affinity for lysosome receptors compared to a therapeutic enzyme to which an Fc is not fused, and thereby can have high duration confirming that such a therapeutic enzyme is effective for the treatment of LSDs.

In an embodiment of the present invention, it was confirmed that the enzyme fusion protein of the present invention, even with a lower administration frequency compared to that of an enzyme to which an Fc region is not fused, reduced the glycosaminoglycan (GAG) value in an IDS-knockout mouse (Example 6).

Additionally, in another embodiment of the present invention, it was confirmed that the enzyme fusion protein of the present invention not only showed a high degree of distribution in the bone marrow and spleen compared to a native enzyme to which an Fc region is not fused, but also showed its distribution in the lungs, kidneys, heart, *etc.,* while the distribution of the native enzyme was not confirmed in the subject tissues (Example 7).

These results suggest that the enzyme fusion protein of the present invention, when administered based on high stability, can not only increase patient convenience by lowering the administration frequency, but can also allow a subcutaneous administration due to its high degree of distribution in tissues.

As used herein, the term "prevention" refers to all activities that inhibit or delay the occurrence of LSD by administering the enzyme fusion protein or composition containing the enzyme fusion protein, and the term "treatment" refers to all activities that improve or advantageously change the symptoms of LSD by administering the enzyme fusion protein or composition containing the enzyme fusion protein.

As used herein, the term "administration" refers to the introduction of a particular substance into a patient by any appropriate method, and the administration route of the composition may be any conventional route that enables delivery of the composition to the target *in vivo,* for example, intraperitoneal administration, intravenous administration, intramuscular administration, subcutaneous administration, intradermal administration, oral administration, local administration, intranasal administration, intrapulmonary administration, intrarectal administration, *etc.* However, since peptides are digested upon oral administration, active ingredients of a composition for oral administration is preferably coated or formulated for protection against degradation in the stomach, and specifically, may be administered in an injectable form. Additionally, the pharmaceutical composition may be administered using a certain device capable of transporting the active ingredients into a target cell.

The total effective dose of the composition of the present invention may be administered to a patient in a single dose or may be administered for a long period of time in multiple doses according to a fractionated treatment protocol. In the pharmaceutical composition of the present invention, the content of the active ingredient may vary depending on the disease severity. Specifically, the total daily dose of the fusion protein of the present invention may be about 0.0001 mg to 500 mg per 1 kg of body weight of a patient. However, the effective dose of the fusion protein is determined considering various factors including the patient's age, body weight, health conditions, sex, disease severity, diet, excretion rate, *etc.* in addition to administration route and treatment frequency of the pharmaceutical composition. In this regard, those skilled in the art may easily determine the effective dose suitable for the particular use of the pharmaceutical composition of the present invention. The pharmaceutical composition according to the present invention is not particularly limited to the formulation, administration route, and method, as long as it shows the effects of the present invention.

In the present invention, the actual dose of the enzyme fusion protein may be determined based on the types of the therapeutic enzyme used as an active ingredient along with various factors such as the disease to be treated, administration route, age, sex, and weight of a patient, severity of the disease, *etc.* Since the enzyme fusion protein of the present invention has significantly excellent *in vivo* duration and activity, the dose, number, and frequency of administration of the pharmaceutical formulation containing the enzyme fusion protein of the present invention can be significantly reduced.

The pharmaceutical composition of the present invention may further contain a pharmaceutically acceptable carrier, excipient, or diluent. The pharmaceutically acceptable carrier may be non-naturally occurring.

As used herein, the term "pharmaceutically acceptable" refers to the properties of having a sufficient amount to exhibit a therapeutic effect and not cause adverse effects, and may be easily determined by those skilled in the art based on factors well known in the medical field, such as the kind of disease, age, weight, health conditions, sex, drug sensitivity of a patient, administration route, administration method, administration frequency, duration of treatment, a drug(s) to be mixed or administered simultaneously, *etc.*

The pharmaceutically acceptable carrier may include, for oral administration, a binder, a glidant, a disintegrant, an excipient, a solubilizing agent, a dispersant, a stabilizing agent, a suspending agent, a coloring agent, a flavoring agent, *etc.;* for injections, a buffering agent, a preserving agent, an analgesic, a solubilizing agent, an isotonic agent, a stabilizing agent, *etc.,* which may be combined to be used; and for topical administrations, a base, an excipient, a lubricant, a preserving agent, *etc.,* but the pharmaceutically acceptable carriers are not limited thereto.

The formulation type of the composition of the present invention may be prepared variously by combining with a pharmaceutically acceptable carrier described above. For example, for oral administration, the composition may be formulated into tablets, troches, capsules, elixirs, suspensions, syrups, wafers, *etc.* For injections, the composition may be formulated into unit-dose ampoules or multi-dose containers. Additionally, the composition may also be formulated into solutions, suspensions, tablets, pills, capsules, sustained-release formulations, *etc.*

Meanwhile, examples of suitable carriers, excipients, and diluents may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anticoagulant, a lubricant, a humectant, a flavoring agent, a preservative, *etc.*

Additionally, the enzyme fusion protein may be used by mixing with various pharmaceutically acceptable carriers approved as pharmaceutical drugs such as physiological saline or organic solvents. For increasing stability or absorptivity, carbohydrates such as glucose, sucrose, or dextrans, and antioxidants such as ascorbic acid or glutathione, chelating agents, low molecular weight proteins, or other stabilizers may be used as pharmaceutical drugs.

The pharmaceutical composition may contain the above ingredients (active ingredients) in an amount of 0.01% to 99% (w/v), but the amount is not limited thereto.

Still another aspect of the present invention provides a polynucleotide encoding the enzyme fusion protein according to the present invention.

The polynucleotide encoding the enzyme fusion protein according to the present invention may be a polynucleotide in a form where a region encoding a therapeutic enzyme and a region encoding a peptide linker-immunoglobulin Fc region is linked, and specifically, a polynucleotide encoding a fusion protein where the N-terminus of an immunoglobulin Fc region is linked to the C-terminus of a therapeutic enzyme through a GGGGS linker, but the polynucleotide is not limited thereto. More specifically, the polynucleotide of the present invention may include the sequence of SEQ ID NO: 1 or 3, but the sequence is not limited thereto as long as the polynucleotide can encode the fusion protein comprising a therapeutic enzyme and an immunoglobulin Fc region.

Still another aspect of the present invention provides a recombinant expression vector including the polynucleotide.

As used herein, the term "recombinant vector" refers to a DNA construct where a target peptide (*e.g*., enzyme fusion protein) is operably linked to an appropriate control sequence to enable the expression of the target peptide (*e.g*., enzyme fusion protein) in an appropriate host. The recombinant vector according to the present invention may be constructed as a vector for typical cloning or as a vector for expression, and may be constructed using a prokaryotic cell or eukaryotic cell as a host cell.

The control sequence includes a promoter capable of initiating transcription, any operator sequence for the control of the transcription, a sequence encoding an appropriate mRNA ribosome-binding domain, and a sequence controlling the termination of transcription and translation. The recombinant vector, after being transformed into a suitable host cell, may be replicated or function irrespective of the host genome, or may be integrated into the host genome itself.

The recombinant vector used in the present invention may not be particularly limited as long as the vector is able to replicate in a host cell, and it may be constructed using any vector known in the art. Examples of the vector may include natural or recombinant plasmids, cosmids, viruses, and bacteriophages. The vector that can be used in the present invention is not particularly limited but any known expression vector may be used.

The recombinant vector is used for the transformation of a host cell for producing the enzyme fusion protein of the present invention. Additionally, these transformed cells, as a part of the present invention, may be used for the amplification of nucleic acid fragments and vectors, or they may be cultured cells or cell lines used in the recombinant production of the enzyme fusion protein of the present invention.

As used herein, the term "transformation" refers to a process of introducing a recombinant vector including a polynucleotide encoding a target protein into a host cell, thereby enabling the expression of the protein encoded by the polynucleotide in the host cell. For the transformed polynucleotide, it does not matter whether it is inserted into the chromosome of a host cell and located therein or located outside the chromosome, as long as it can be expressed in the host cell, and both cases are included.

Additionally, the polynucleotide includes DNA and RNA which encode the target protein. The polynucleotide may be inserted in any form as long as it can be introduced into a host cell and expressed therein. For example, the polynucleotide may be introduced into a host cell in the form of an expression cassette, which is a gene construct including all essential elements required for self-expression. The expression cassette may conventionally include a promoter operably linked to the polynucleotide, a transcription termination signal, a ribosome-binding domain, and a translation termination signal. The expression cassette may be in the form of an expression vector capable of self-replication. Additionally, the polynucleotide may be introduced into a host cell as is and operably linked to a sequence essential for its expression in the host cell, but the polynucleotide is not limited thereto.

Additionally, as used herein, the term "operably linked" refers to a functional linkage between a promoter sequence, which initiates and mediates the transcription of the polynucleotide encoding the target peptide of the present invention, and the above gene sequence.

An appropriate host to be used in the present invention may not be particularly limited as long as it can express the polynucleotide of the present invention. Examples of the appropriate host may include bacteria belonging to the genus *Escherichia* such as *E. coli;* bacteria belonging to the genus *Bacillus* such as *Bacillus subtilis;* bacteria belonging to the genus *Pseudomonas* such as *Pseudomonas putida;* yeasts such as *Pichia pastoris, Saccharomyces cerevisiae,* and *Schizosaccharomyces pombe;* insect cells such as *Spodoptera frugiperda* (Sf9); and animal cells such as CHO, COS, BSC, *etc.*

Still another aspect of the present invention provides a transformant into which the expression vector is introduced.

For the purpose of the present invention, the transformant into which the expression vector of the present invention is introduced may not be limited as long as the transformant can express and produce the enzyme fusion protein, but the transformant may be bacteria belonging to the genus *Escherichia* such as *E. coli;* bacteria belonging to the genus *Bacillus* such as *Bacillus subtilis;* bacteria belonging to the genus *Pseudomonas* such as *Pseudomonas putida;* yeasts such as *Pichia pastoris, Saccharomyces cerevisiae,* and *Schizosaccharomyces pombe;* insect cells such as *Spodoptera frugiperda* (Sf9); and animal cells such as CHO, COS, BSC, *etc.*

Still another aspect of the present invention provides a method for preparing the enzyme fusion protein according to the present invention.

Specifically, the method may include (a) culturing a transformant to obtain a culture; and (b) recovering an enzyme fusion protein from the culture, but the method is not limited thereto.

In the present invention, the medium used in culturing the transformant must meet the requirements for host cell cultivation in an appropriate manner. The carbon sources that may be contained in the medium for the growth of a host cell may be appropriately selected by the decision of those skilled in the art according to the type of the transformant prepared thereof, and appropriate cultivation conditions may be selected so as to control the period and amount of cultivation.

Examples of the sugar source to be used in the medium may include sugars and carbohydrates such as glucose, saccharose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These materials may be used alone or in combination.

Examples of the nitrogen source to be used may include peptone, yeast extract, meat gravy, malt extract, corn steep liquor, soybean flour, and urea, or inorganic compounds such as ammonium sulfate, ammonium chloride, ammonium phosphate, ammonium carbonate, and ammonium nitrate. The nitrogen source may also be used alone or in combination.

Examples of the phosphorous source to be used may include potassium dihydrogen phosphate or dipotassium hydrogen phosphate or a corresponding sodium-containing salt. Additionally, the culture medium may contain a metal salt such as magnesium sulfate or iron sulfate necessary for growth.

Lastly, essential growth materials such as amino acids and vitamins may be used. Additionally, appropriate precursors for culture medium may also be used. The above sources may be appropriately added to a culture during cultivation by a batch culture or continuous culture. The pH of the culture may be appropriately adjusted using a basic compound such as sodium hydroxide, potassium hydroxide, and ammonia, or an acid compound such as phosphoric acid or sulfuric acid. Additionally, an antifoaming agent such as fatty acid polyglycol ester may be added to prevent foam generation. Additionally, in order to maintain the aerobic state of the culture, oxygen or an oxygen-containing gas (*e.g.,* air) may be injected into the culture.

The transformant of the present invention may be cultured at 20°C to 45°C, and specifically, 25°C to 40°C. Additionally, the cultivation is continued until the maximum amount of production of the desired enzyme fusion protein is obtained, and in this regard, the cultivation may normally be continued for 10 hours to 160 hours.

As described above, the transformant of the present invention can produce enzyme fusion protein when appropriate culture conditions are provided according to a host cell, and the enzyme fusion protein produced according to the vector constitution and characteristics of a host cell may be secreted within the cytoplasm or into the periplasmic space of the host cell or extracellularly.

The proteins expressed within or outside of the host cell may be purified by a conventional method. Examples of the purification method may include salting-out (*e.g*., ammonium sulfate precipitation, sodium phosphate precipitation, *etc.*)*,* solvent precipitation (*e.g.,* protein fraction precipitation using acetone or ethanol, *etc.*)*,* dialysis, gel filtration, ion exchange, or chromatography such as reversed column chromatography, ultrafiltration, *etc.,* and these methods may be used alone or in combination.

Still another aspect of the present invention provides a method for the prevention or treatment of LSD to a subject, including administering the enzyme fusion protein or a composition containing the enzyme fusion protein.

Since the enzyme fusion protein of the present invention contains a therapeutic enzyme which can prevent or treat LSD, a subject which is suspected of having the LSD may be prevented or treated by the administration of an enzyme fusion protein containing the therapeutic enzyme or a pharmaceutical composition containing the enzyme fusion protein.

As used herein, the term "subject" refers to a subject suspected of having LSD, and the subject suspected of having LSD refers to mammals including humans, rats, cattle, *etc.,* which have or are at risk of developing the LSD, but any subject which can be treated with the enzyme fusion protein of the present invention or composition containing the enzyme fusion protein is included without limitation.

The method of the present invention may include administering a pharmaceutically effective amount of the pharmaceutical composition containing an enzyme fusion protein. An appropriate total daily dose of the composition may be determined within the scope of correct medical judgment by a practitioner, and the composition may be administered once or several times in divided doses. However, for the purpose of the present invention, preferably, the specific therapeutically effective dose of the composition for any particular patient is applied differently depending on various factors including the kind and degree of responses to be achieved, specific compositions including whether other agents are occasionally used therewith, the patient's age, weight, health conditions, sex and diet, administration time, administration route, excretion rate of the composition, duration of treatment, other drugs used in combination or simultaneously with the specific compositions, and similar factors well known in the medical field.

Meanwhile, the method for the prevention or treatment of the LSD may be a combination therapy which further includes administering a compound or material having a therapeutic effect for at least one of the LSDs, but the method is not limited thereto.

As used herein, the term "combination" must be understood as referring to a simultaneous, separate, or sequential administration. When the administration is sequential or separate, the interval allowed for the administration of a second ingredient must be one which should not lose the advantageous effects of the combination.

The administration dose of the enzyme fusion protein having a therapeutic activity for the LSD may be about 0.0001 µg to 500 mg per 1 kg of body weight of a patient, but the dose is not particularly limited.

Still another aspect of the present invention provides a use of the enzyme fusion protein, or a composition containing the enzyme fusion protein in the preparation of a medicament (or a pharmaceutical composition) for the prevention or treatment of LSDs.

Hereinafter, the present invention will be described in more detail with reference to the following Examples. However, these Examples are for illustrative purposes only and the scope of the invention is not limited by these Examples.

### Example 1: Preparation of expression vector for fusion protein

For the production of enzyme fusion proteins, an expression vector for fusion proteins was prepared by overlapping PCR using an expression vector (IDS cDNA, Cat No. EX-C0003-M02, Gencopoeia; ARSB cDNA, Cat No. EX-C0073-M02, Genecopoeia), where naturally occurring iduronate-2-sulfatase (IDS, SEQ ID NO: 1) and arylsulfatase B (ARSB, SEQ ID NO: 3) are inserted, respectively, a synthesized linker (SEQ ID NO: 5), and an IgG4 Fc region (SEQ ID NO: 7). Since the overlapping PCR technique includes sequences that overlap with the primers when amplifying each of the enzyme and the linker-Fc, the produced PCR products will include the overlapping sequences. For the amplification of the fusion protein, PCR was performed as follows: 1) primary PCR (25 cycles consisting of 95°C for 1 min; 57°C for 30 sec; and 68°C for 3 min) and 2) secondary PCR (25 cycles consisting of 95°C for 1 min; 57°C for 30 sec; and 68°C for 4 min).

Specifically, for IDS, PCR was performed using the primers of SEQ ID NOS: 10 and 11; and for the linker-Fc, PCR was performed using the primers of SEQ ID NOS: 12 and 13. As a result, the IDS PCR product included the linker-Fc sequence at the 3' end and the linker-Fc PCR included the IDS sequence at the 5' end.

The secondary PCR was performed using the two PCR products obtained in the primary PCR as templates along with the primers (SEQ ID NOS: 10 and 13) and then the PCR product having the IDS-Fc sequence was obtained. The overlapping sequence in the product having the IDS-Fc sequence was digested with restriction enzymes (*Kpn*I and *Xho*I) and the resulting PCR product was inserted into the X0GC vector to prepare an expression vector (pX0GC-Enzyme-Fc).

In the same manner, a PCR product having the ARSB-Fc sequence was obtained using the primers (SEQ ID NOS: 14, 15, 16, and 17). The resulting PCR product was digested with the restriction enzymes (*Kpn*I and *Xho*I) and inserted with the X0GC vector, which was already digested with the same restriction enzymes (*Kpn*I and *Xho*I)*,* to prepare an expression vector for fusion proteins.

**[Table 1] Overlapping PCR primer**

| | Sequence | SEQ ID NO |
|---|---|---|
| IDS-F (*Kpn*I) | 5'-CAGGTACCATGCCGCCACCCCGGACC-3' | 10 |
| IDS-R (overlap) | 5′- TGAACCGCCTCCACCAGGCATCAACAACTGGAAAAGAT CTCCAC-3′ | 11 |
| L15Fc (IDS)-F | 5'-CAGTTGTTGATGCCTGGTGGAGGCGGTTCAGGCG-3' | 12 |
| L15Fc-R (*Xho*I) | 5'-GACTCGAGTCATTTACCCAGAGACAGGGAGAGG-3' | 13 |
| ARSB-F (*Kpn*I) | 5'-CAGGTACCATGGGTCCGCGCGGCGCG-3' | 14 |
| ARSB-R (overlap) | 5'-TGAACCGCCTCCACCCATCCAAGGGCCCCACACCC-3' | 15 |
| L15Fc(ARSB)-F | 5'-TGGGGCCCTTGGATGGGTGGAGGCGGTTCAGGCG-3' | 16 |
| L15Fc-R (*Xho*I) | 5'-GACTCGAGTCATTTACCCAGAGACAGGGAGAGG-3' | 17 |

The chain exchange and the N-glycosylation site in the Fe region of the sequences of the prepared fusion proteins were removed by the site-directed mutagenesis PCR technique.

Specifically, the 2^{nd} amino acid of the Fc region (*i.e.,* serine) involved in the chain exchange was substituted with proline using the primers (SEQ ID NOS: 18 and 19), and the 71^{st} amino acid of the Fc region (*i.e.,* asparagine) involved in the N-glycosylation was substituted with glutamine. In the protein sequences shown in Table 3 below, each of the letters in bold indicates that the subject amino acid was substituted and those in italic indicate linkers.

**[Table 2] Mutagenesis primer**

| Primer | Sequence | SEQ ID NO |
|---|---|---|
| Fc(S2P)_F | 5′- CTGGCGGTGGCGGATCGCCACCATGCCCAGCACCTGAGT TCCT-3′ | 18 |
| Fc(S2P)_R | 5′- AGGAACTCAGGTGCTGGGCATGGTGGCGATCCGCCACCG CCAG-3′ | 19 |
| Fc(N71Q) F | 5′- AGCCGCGGGAGGAGCAGTTCCAAAGCACGTACCGTGTGG TCAG-3′ | 20 |
| Fc(N71Q) R | 5′- CTGACCACACGGTACGTGCTTTGGAACTGCTCCTCCCGCG GCT-3′ | 21 |

The expression vectors for enzyme fusion proteins prepared in Examples above were named as IDS-Fc vector and ARSB-Fc vector, respectively. Alternatively, these vectors may be used interchangeably with the pXOGC-Enzyme-Fc.

**[Table 3] Enzyme fusion protein DNA sequence and protein sequence**

| Name | Sequence | | SEQ ID NO |
|---|---|---|---|
| IDS-Fc | DNA | ATGCCGCCACCCCGGACCGGCCGAGGCCTTCTCTG GCTGGGTCTG GTTCTGAGCT CCGTCTGCGT CGCCCTCGGA TCCGAAACGC AGGCCAACTC GACCACAGAT GCTCTGAACG TTCTTCTCAT CATCGTGGAT GACCTGCGCC CCTCCCTGGG CTGTTATGGG GATAAGCTGG TGAGGTCCCC AAATATTGAC CAACTGGCAT | 22 |
| | | CCCACAGCCT CCTCTTCCAG AATGCCTTTG CGCAGCAAGC AGTGTGCGCC CCGAGCCGCG TTTCTTTCCT CACTGGCAGG AGACCTGACA CCACCCGCCT GTACGACTTC AACTCCTACT GGAGGGTGCA CGCTGGAAAC TTCTCCACCA TCCCCCAGTA CTTCAAGGAG AATGGCTATG TGACCATGTC GGTGGGAAAA GTCTTTCACC CTGGGATATC TTCTAACCAT ACCGATGATT CTCCGTATAG CTGGTCTTTT CCACCTTATC ATCCTTCCTC TGAGAAGTAT GAAAACACTA AGACATGTCG AGGGCCAGAT GGAGAACTCC ATGCCAACCT GCTTTGCCCT GTGGATGTGC TGGATGTTCC CGAGGGCACC TTGCCTGACA AACAGAGCAC TGAGCAAGCC ATACAGTTGT TGGAAAAGAT GAAAACGTCA GCCAGTCCTT TCTTCCTGGC CGTTGGGTAT CATAAGCCAC ACATCCCCTT CAGATACCCC AAGGAATTTC AGAAGTTGTA TCCCTTGGAG AACATCACCC TGGCCCCCGA TCCCGAGGTC CCTGATGGCC TACCCCCTGT GGCCTACAAC CCCTGGATGG ACATCAGGCA ACGGGAAGAC GTCCAAGCCT TAAACATCAG TGTGCCGTAT GGTCCAATTC CTGTGGACTT TCAGCGGAAA ATCCGCCAGA GCTACTTTGC CTCTGTGTCA TATTTGGATA CACAGGTCGG CCGCCTCTTG AGTGCTTTGG ACGATCTTCA GCTGGCCAAC AGCACCATCA TTGCATTTAC CTCGGATCAT GGGTGGGCTC TAGGTGAACA TGGAGAATGG GCCAAATACA GCAATTTTGA TGTTGCTACC CATGTTCCCC TGATATTCTA TGTTCCTGGA AGGACGGCTT CACTTCCGGA GGCAGGCGAG AAGCTTTTCC CTTACCTCGA CCCTTTTGAT TCCGCCTCAC AGTTGATGGA GCCAGGCAGG CAATCCATGG ACCTTGTGGA ACTTGTGTCT CTTTTTCCCA CGCTGGCTGG ACTTGCAGGA CTGCAGGTTC CACCTCGCTG CCCCGTTCCT TCATTTCACG TTGAGCTGTG CAGAGAAGGC AAGAACCTTC TGAAGCATTT TCGATTCCGT GACTTGGAAG AGGATCCGTA CCTCCCTGGT AATCCCCGTG AACTGATTGC CTATAGCCAG TATCCCCGGC CTTCAGACAT CCCTCAGTGG AATTCTGACA AGCCGAGTTT AAAAGATATA AAGATCATGG GCTATTCCAT ACGCACCATA GACTATAGGT ATACTGTGTG GGTTGGCTTC AATCCTGATG AATTTCTAGC TAACTTTTCT GACATCCATG CAGGGGAACT GTATTTTGTG GATTCTGACC CATTGCAGGA TCACAATATG TATAATGATT CCCAAGGTGG AGATCTTTTC CAGTTGTTGA | |
| | | TGCCTGGTGG AGGCGGTTCA GGCGGAGGTG GCTCTGGCGG TGGCGGATCG CCATCATGCC CAGCACCTGA GTTCCTGGGG GGACCATCAG TCTTCCTGTT CCCCCCAAAA CCCAAGGACA CCCTCATGAT CTCCCGGACC CCTGAGGTCA CATGCGTGGT GGTGGACGTG AGCCAGGAAG ACCCTGAGGT CCAGTTCAAC TGGTACGTGG ACGGCGTGGA GGTGCATAAT GCCAAGACAA AGCCGCGGGA GGAGCAGTTC AACAGCACGT ACCGTGTGGT CAGCGTCCTC ACCGTCCTGC ACCAGGACTG GCTGAATGGC AAGGAGTACA AGTGCAAGGT CTCCAACAAA GGCCTCCCAT CCTCCATCGA GAAAACCATC TCCAAAGCCA AAGGGCAGCC CCGAGAACCA CAGGTGTACA CCCTGCCCCC ATCCCAGGAG GAGATGACCA AGAACCAGGT CAGCCTGACC TGCCTGGTCA AAGGCTTCTA TCCCAGCGAC ATCGCCGTGG AGTGGGAGAG CAATGGGCAG CCGGAGAACA ACTACAAGAC CACGCCTCCC GTGCTGGACT CCGACGGCTC CTTCTTCCTC TACAGCAGGC TAACCGTGGA CAAGAGCAGG TGGCAGGAGG GGAACGTCTT CTCATGCTCC GTGATGCATG AGGCTCTGCA CAACCACTAC ACGCAGAAGA GCCTCTCCCT GTCTCTGGGT AAATGA | |
| | Protein | MPPPR TGRGLLWLGL VLSSVCVALG SETQANSTTD ALNVLLIIVD DLRPSLGCYG DKLVRSPNID QLASHSLLFQ NAFAQQAVCA PSRVSFLTGR RPDTTRLYDF NSYWRVHAGN FSTIPQYFKE NGYVTMSVGK VFHPGISSNH TDDSPYSWSF PPYHPSSEKY ENTKTCRGPD GELHANLLCP VDVLDVPEGT LPDKQSTEQA IQLLEKMKTS ASPFFLAVGY HKPHIPFRYP KEFQKLYPLE NITLAPDPEV PDGLPPVAYN PWMDIRQRED VQALNISVPY GPIPVDFQRK IRQSYFASVS YLDTQVGRLL SALDDLQLAN STIIAFTSDH GWALGEHGEW AKYSNFDVAT HVPLIFYVPG RTASLPEAGE KLFPYLDPFD SASQLMEPGR QSMDLVELVS LFPTLAGLAG LQVPPRCPVP SFHVELCREG KNLLKHFRFR DLEEDPYLPG NPRELIAYSQ YPRPSDIPQW NSDKPSLKDI KIMGYSIRTI DYRYTVWVGF NPDEFLANFS DIHAGELYFV DSDPLQDHNM YNDSQGGDLF QLLMPGGGGS GGGGSGGGGS PPCPAPEFLG GPSVFLFPPK PKDTLMISRT PEVTCVVVDV SQEDPEVQFN WYVDGVEVHN AKTKPREEQF QSTYRVVSVL TVLHQDWLNG KEYKCKVSNK GLPSSIEKTI SKAKGQPREP QVYTLPPSQE EMTKNQVSLT CLVKGFYPSD IAVEWESNGQ | 23 |
| | | PENNYKTTPP VLDSDGSFFL YSRLTVDKSR WQEGNVFSCS VMHEALHNHY TQKSLSLSLG K | |
| ARSB-Fc | DNA | ATGGGTCC GCGCGGCGCG GCGAGCTTGC CCCGAGGCCC CGGTCCTCGG CGGCTGCTTC TCCCCGTCGT CCTCCCGCTG CTGCTGCTGC TGTTGTTGGC GCCGCCGGGC TCGGGCGCCG GGGCCAGCCG GCCGCCCCAC CTGGTCTTCT TGCTGGCAGA CGACCTAGGC TGGAACGACG TCGGCTTCCA CGGCTCCCGC ATCCGCACGC CGCACCTGGA CGCGCTGGCG GCCGGCGGGG TGCTCCTGGA CAACTACTAC ACGCAGCCGC TGTGCACGCC GTCGCGGAGC CAGCTGCTCA CTGGCCGCTA CCAGATCCGT ACAGGTTTAC AGCACCAAAT AATCTGGCCC TGTCAGCCCA GCTGTGTTCC TCTGGATGAA AAACTCCTGC CCCAGCTCCT AAAAGAAGCA GGTTATACTA CCCATATGGT CGGAAAATGG CACCTGGGAA TGTACCGGAA AGAATGCCTT CCAACCCGCC GAGGATTTGA TACCTACTTT GGATATCTCC TGGGTAGTGA AGATTATTAT TCCCATGAAC GCTGTACATT AATTGACGCT CTGAATGTCA CACGATGTGC TCTTGATTTT CGAGATGGCG AAGAAGTTGC AACAGGATAT AAAAATATGT ATTCAACAAA CATATTCACC AAAAGGGCTA TAGCCCTCAT AACTAACCAT CCACCAGAGA AGCCTCTGTT TCTCTACCTT GCTCTCCAGT CTGTGCATGA GCCCCTTCAG GTCCCTGAGG AATACTTGAA GCCATATGAC TTTATCCAAG ACAAGAACAG GCATCACTAT GCAGGAATGG TGTCCCTTAT GGATGAAGCA GTAGGAAATG TCACTGCAGC TTTAAAAAGC AGTGGGCTCT GGAACAACAC GGTGTTCATC TTTTCTACAG ATAACGGAGG GCAGACTTTG GCAGGGGGTA ATAACTGGCC CCTTCGAGGA AGAAAATGGA GCCTGTGGGA AGGAGGCGTC CGAGGGGTGG GCTTTGTGGC AAGCCCCTTG CTGAAGCAGA AGGGCGTGAA GAACCGGGAG CTCATCCACA TCTCTGACTG GCTGCCAACA CTCGTGAAGC TGGCCAGGGG ACACACCAAT GGCACAAAGC CTCTGGATGG CTTCGACGTG TGGAAAACCA TCAGTGAAGG AAGCCCATCC CCCAGAATTG AGCTACTGCA TAATATTGAC CCGAACTTCG TGGACTCTTC ACCGTGTCCC AGGAACAGCA TGGCTCCAGC AAAGGATGAC TCTTCTCTTC CAGAATATTC AGCCTTTAAC ACATCTGTCC ATGCTGCAAT TAGACATGGA AATTGGAAAC TCCTCACGGG CTACCCAGGC TGTGGTTACT GGTTCCCTCC | 24 |
| | | ACCGTCTCAA TACAATGTTT CTGAGATACC CTCATCAGAC CCACCAACCA AGACCCTCTG GCTCTTTGAT ATTGATCGGG ACCCTGAAGA AAGACATGAC CTGTCCAGAG AATATCCTCA CATCGTCACA AAGCTCCTGT CCCGCCTACA GTTCTACCAT AAACACTCAG TCCCCGTGTA CTTCCCTGCA CAGGACCCCC GCTGTGATCC CAAGGCCACT GGGGTGTGGG GCCCTTGGAT GGGTGGAGGC GGTTCAGGCG GAGGTGGCTC TGGCGGTGGC GGATCGCCAT CATGCCCAGC ACCTGAGTTC CTGGGGGGAC CATCAGTCTT CCTGTTCCCC CCAAAACCCA AGGACACCCT CATGATCTCC CGGACCCCTG AGGTCACATG CGTGGTGGTG GACGTGAGCC AGGAAGACCC TGAGGTCCAG TTCAACTGGT ACGTGGACGG CGTGGAGGTG CATAATGCCA AGACAAAGCC GCGGGAGGAG CAGTTCAACA GCACGTACCG TGTGGTCAGC GTCCTCACCG TCCTGCACCA GGACTGGCTG AATGGCAAGG AGTACAAGTG CAAGGTCTCC AACAAAGGCC TCCCATCCTC CATCGAGAAA ACCATCTCCA AAGCCAAAGG GCAGCCCCGA GAACCACAGG TGTACACCCT GCCCCCATCC CAGGAGGAGA TGACCAAGAA CCAGGTCAGC CTGACCTGCC TGGTCAAAGG CTTCTATCCC AGCGACATCG CCGTGGAGTG GGAGAGCAAT GGGCAGCCGG AGAACAACTA CAAGACCACG CCTCCCGTGC TGGACTCCGA CGGCTCCTTC TTCCTCTACA GCAGGCTAAC CGTGGACAAG AGCAGGTGGC AGGAGGGGAA CGTCTTCTCA TGCTCCGTGA TGCATGAGGC TCTGCACAAC CACTACACGC AGAAGAGCCT CTCCCTGTCT CTGGGTAAAT GA | |
| | Protein | MGPRGA ASLPRGPGPR RLLLPVVLPL LLLLLLAPPG SGAGASRPPH LVFLLADDLG WNDVGFHGSR IRTPHLDALA AGGVLLDNYY TQPLCTPSRS QLLTGRYQIR TGLQHQIIWP CQPSCVPLDE KLLPQLLKEA GYTTHMVGKW HLGMYRKECL PTRRGFDTYF GYLLGSEDYY SHERCTLIDA LNVTRCALDF RDGEEVATGY KNMYSTNIFT KRAIALITNH PPEKPLFLYL ALQSVHEPLQ VPEEYLKPYD FIQDKNRHHY AGMVSLMDEA VGNVTAALKS SGLWNNTVFI FSTDNGGQTL AGGNNWPLRG RKWSLWEGGV RGVGFVASPL LKQKGVKNRE LIHISDWLPT LVKLARGHTN GTKPLDGFDV WKTISEGSPS PRIELLHNID PNFVDSSPCP RNSMAPAKDD SSLPEYSAFN TSVHAAIRHG NWKLLTGYPG CGYWFPPPSQ YNVSEIPSSD PPTKTLWLFD IDRDPEERHD | 25 |
| | | LSREYPHIVT KLLSRLQFYH KHSVPVYFPA QDPRCDPKAT GVWGPWMGGG GSGGGGSGGG GSPPCPAPEF LGGPSVFLFP PKPKDTLMIS RTPEVTCVVV DVSQEDPEVQ FNWYVDGVEV HNAKTKPREE QFQSTYRVVS VLTVLHQDWL NGKEYKCKVS NKGLPSSIEK TISKAKGQPR EPQVYTLPPS QEEMTKNQVS LTCLVKGFYP SDIAVEWESN GQPENNYKTT PPVLDSDGSF FLYSRLTVDK SRWQEGNVFS CSVMHEALHN HYTQKSLSLS LGK | |

### Example 2: Transformation of CHO cell line using expression vector for fusion protein

The recombinant expression vector pXOGC-Enzyme-Fc prepared in Example 1 was introduced into the *DG44*/*CHO* cell line (CHO/dhfr-) (Urlaub et al., Somat. Cell. Mol. Genet., 12, 555 to 566, 1986), in which the *DHFR* gene is damaged and thus its biosynthesis process of nucleic acid is imperfect, to obtain a transformant, and the enzyme fusion protein (Enzyme-Fc) was expressed in the transformant.

Specifically, the *DG44*/*CHO* cell line was cultured to a confluence such that the cells cover about 80% to about 90% of the bottom of the container, and the cells were washed 3 times with Opti-MEM (Gibco, Cat. No. 51985034).

Meanwhile, a mixture of Opti-MEM (3 mL) and pXOGC-Enzyme-Fc (an expression vector, 5 µg) and a mixture of Opti-MEM (3 mL) and lipofectamine 2000 (Gibco, Cat. No. 11668-019, 20 µL) were placed at room temperature for 30 minutes. Then, the two mixtures were mixed together and the cultured *DG44*/*CHO* cell line was added thereto and cultured at 37°C and 5% CO₂ conditions for about 18 hours to introduce the pXOGC-Enzyme-Fc expression vector into the *DG44*/*CHO* cell line.

Then, the cultured cells were washed 3 times with DMEM-F12 medium containing 10% FBS (Gibco, Cat. No. 11330) and the medium was added thereto and cultured again for 48 hours. Trypsin was added to the cultured cells to separate the cultured cells, and these separated cells were inoculated into a selection medium (the α-MEM medium (WELGENE, Cat. No. LM008-02) which did not contain HT supplement (hypoxanthine-thymidine) but contained 10% FBS and 1 mg/mL of G418 (Cellgro, Cat. No. 61-234-RG)). Transformed cells were selected from the selection medium by culturing the cells while replacing the medium at intervals of 2 days or 3 days until only the transformed cells survived and formed colonies. In particular, for the improvement of the expression levels of the enzyme fusion proteins in the selected transformed cells, 10 nM MTX (Sigma, Cat. No. M8407) was added to the selection medium and the concentrations were gradually increased, and thereby the MTX amounts of these transformed cells were increased to 20 nM one to two weeks thereafter.

### Example 3: Confirmation of expression of IDS-Fc, ARSB-Fc fusion proteins by ELISA

Part of the transformed cells prepared in Example 2 were transferred into each of the 175-T cell culture flasks at a concentration 1 × 10⁷ cells and cultured until the cells almost covered the bottom of the culture container, and then 15 mL of serum-free Ex-cell medium (purchased from Sigma by custom order, Cat. No. 14360C) charged with 1 mM sodium butyrate (Sigma, Cat. No. B5887) was added to each flask, and these were cultured in an incubator (33°C, 5% CO₂) for 48 hours. Each cell culture was transferred to a 50 mL tube, centrifuged, and the supernatants were collected again and the expression levels of the fusion proteins (IDS-Fc and ARSB-Fc) were measured.

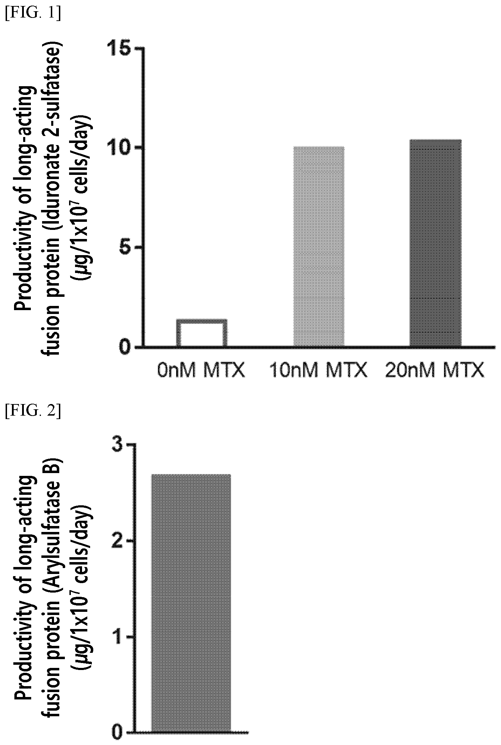

First, the expression level of the IDS-Fc was performed by applying the indirect ELISA method. Human *alpha*-IDS antibodies (R&D Systems, Cat. No. AF2449) diluted in PBS at a concentration of 1 µg/mL were added to a 96-well ELISA plate (Nunk, Cat. No. 44-2404-21) in an amount of 100 µL/well and were reacted in a refrigerator (4°C) overnight. On the following day, the resultant was washed 5 times with PBS-T buffer, and the culture samples and the IDS standard product (Shire Pharmaceuticals Group, Elaprase®, Lot No. TEPE09A17), which was diluted at various concentrations, were each dispensed in an amount of 100 µL/well, and reacted at room temperature for one hour. After one hour, the plate was washed and biotin-labeled human *alpha*-IDS antibodies (R&D Systems, Cat. No. BAF 2449) were added thereto and the mixture was reacted at room temperature for one hour. Lastly, streptavidin-HRP (GE Healthcare, Cat. No. RPN440IV) was diluted in a 1:30,000 ratio and the diluted mixture was added in an amount of 100 µL/well, and reacted for one hour. The resultant was washed and a substrate solution was added thereto and reacted for about 10 minutes. After stopping the reaction with a reaction-stopping solution, the absorbance of the resultant was measured at 450 nm. After obtaining standard curves and functions using the concentrations of the human IDS standard product and absorbance values, the amounts of the human IDS-Fc fusion proteins were quantified. As a result, it was confirmed that the human IDS-Fc fusion protein was expressed in a certain amount from the selected transformed cells (FIG. 1).

Additionally, the expression level of the ARSB-Fc fusion protein was measured by the enzyme immunoassay (Bethyl, Cat No. E80-104) that can quantify human IgG. The human IgG-Fc antibodies (Bethyl, Cat. No. A80-104A-9), which were diluted at a concentration of 10 µg/mL in a carbonate buffer (0.05 M carbonate-bicarbonate, pH 9.6), were added to a 96-well ELISA plate (Nunk, Cat. No. 44-2404-21) in an amount of 100 µL/well, and was reacted at room temperature for one hour. After one hour, the ELISA plate was washed 5 times with a washing solution, and each of the culture samples and the Human IgG standard product included in the human IgG quantification kit (Bethyl, Cat. No. RS10-110-4) were diluted at various concentrations and each dispensed in an amount of 100 µL/well, and reacted at room temperature for one hour. After one hour, the plate was washed and HRP-labeled human IgG-Fc antibodies (Bethyl, Cat. No. A80-104P-87) diluted in a 1:150,000 ratio were added thereto, and reacted at room temperature for one hour. Lastly, streptavidin-HRP (GE Healthcare, Cat. No. RPN440IV) was diluted in a 1:30,000 ratio and was added in an amount of 100 µL/well, and reacted for one hour. The resultant was washed and a substrate solution was added thereto and reacted for about 15 minutes. After stopping the reaction with a reaction-stopping solution, the absorbance of the resultant was measured at 450 nm.

After obtaining standard curves and functions using the concentrations of the human IgG standard product and absorbance values, the amounts of the human ARSB-Fc fusion proteins were quantified. As a result, it was confirmed that the human ARSB-Fc fusion protein was expressed in a certain amount from the selected transformed cells (FIG. 2).

### Example 4: Confirmation of pharmacokinetics of long-acting enzyme fusion protein

The effects of preparation of fusion proteins were compared by examining the pharmacokinetics of the long-acting enzyme fusion proteins prepared above and the enzyme to which an Fc region is not fused.

### Example 4-1: Experiment on pharmacokinetics of long-acting iduronate-2-sulfatase fusion protein

The present inventors made an attempt to confirm the therapeutic duration of the fusion proteins of the present invention by examining the pharmacokinetics of the long-acting fusion protein of iduronate-2-sulfatase prepared in Examples above.

For this purpose, iduronate-2-sulfatase (idursulfase, control group) and the long-acting fusion protein of iduronate-2-sulfatase (IDS-Fc fusion protein, experimental group) were administered to 3 ICR mice, respectively, and the stability in blood and pharmacokinetic coefficients per blood sample collection according to each group were compared.

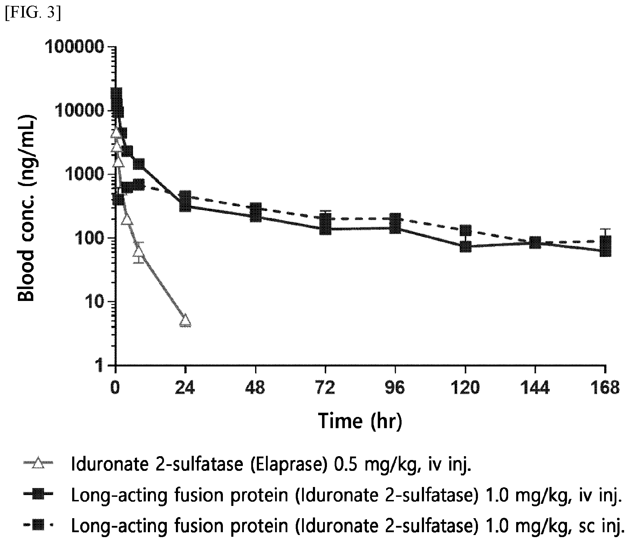

Specifically, based on concentration of iduronate-2-sulfatase, the proteins were administered to the ICR mice of the control group and the experimental group by intravenous and subcutaneous injections at concentrations of 0.5 mg/kg and 1.0 mg/kg, respectively. Blood samples were collected from the group administered by intravenous injection at 0, 0.25, 0.5, 1, 2, 4, 8, 24, 48, 72, 96, 120, 144, and 168 hours after the injection, and from the group administered by subcutaneous injection at 0, 1, 4, 8, 24, 48, 72, 96, 120, 144, 168, 192, and 216 hours after the injection. The amounts of proteins in the blood serum were measured using human specific anti-iduronate-2-sulfatase antibodies by the ELISA method. The analysis results are shown in FIG. 3 and Table 4.

**[Table 4]**

| PK Profile | IDS | IDS-Fc fusion protein | |
|---|---|---|---|
| Administration Conc. | 0.5 mg/kg (IV) | 1.0 mg/kg, IV | 1.0 mg/kg, SC |
| Degree of *in vivo* exposure (ng/mL*hr) | 6047.0 | 67766.8 | 43974.4 |
| Maximum Drug Conc. in Blood (ng/mL) | 67670.8 | 28592.2 | 687.7 |
| Blood Half-Life (hr) | 4.4 | NA | 45.3 |
| *In vivo* Bioavailability (%) | - | - | **64.9** |

As can be seen in the above results, the long-acting fusion protein of iduronate-2-sulfatase according to the present invention showed significantly excellent pharmacokinetic characteristics compared to those of the control group. These results suggest that the long-acting fusion protein of iduronate-2-sulfatase of the present invention has the advantage of reducing the intervals of drug administration in the actual administration of the drug through the long-acting effect compared to enzymes which are not long-acting fusion proteins.

As can be seen in the results of pharmacokinetics of FIG. 3 and Table 4, in the case of the long-acting fusion protein of iduronate-2-sulfatase, all of the half-life (T_{1/2}), maximum drug concentration in blood (Cₘₐₓ), and *in vivo* bioavailability (AUC) were increased. In particular, the *in vivo* bioavailability of the long-acting fusion protein of iduronate-2-sulfatase was 64.9%, thus showing excellent *in vivo* bioavailability compared to enzymes which are not long-acting fusion proteins.

### Example 4-2: Experiment on pharmacokinetics of long-acting arylsulfatase B fusion protein

The present inventors made an attempt to examine the pharmacokinetics of the long-acting fusion protein of arylsulfatase B (ARSB-Fc fusion protein) prepared in Examples above, and as such, measured the pharmacokinetics of the long-acting fusion protein of arylsulfatase B and compared the results with those of arylsulfatase B.

Specifically, based on concentration of arylsulfatase B, the proteins were administered to the ICR mice of the control group (naturally occurring arylsulfatase B: Naglazyme®, BioMarin) and the experimental group (long-acting fusion protein of arylsulfatase B) by intravenous and subcutaneous injections at a concentration of 5.0 mg/kg each. Blood samples were collected from the control group at 0, 0.25, 0.5, 0.75, 1, 1.5, 4, 8, and 24 hours after the injection regardless of the administration method. In the experimental group, from the ICR mice administered by intravenous injection, blood samples were collected at 0, 0.25, 0.5, 1, 1.5, 2, 4, 8, 24, 48, 96, and 168 hours after the injection, and from those administered by subcutaneous injection, blood samples were collected at 0, 0.5, 1, 2, 4, 8, 24, 48, 96, and 168 hours after the injection.

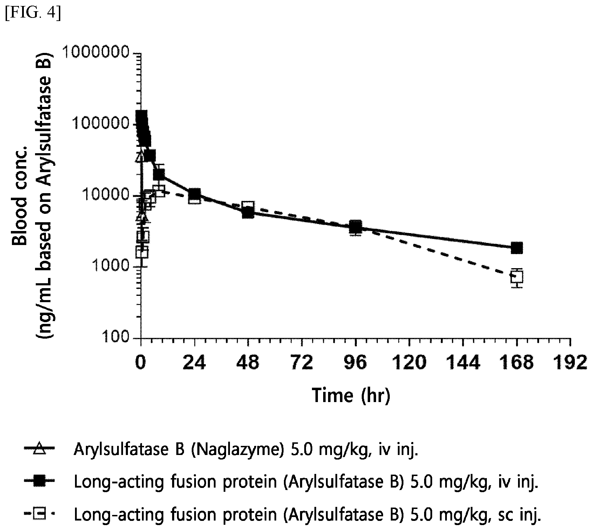

The collected blood samples in each group were centrifuged and separated into sera, and the amounts of the long-acting fusion protein of arylsulfatase B and the naturally occurring arylsulfatase B in the blood were quantified by the enzyme immunoassay method, and the analysis results are shown in FIG. 4 and Table 5.

**[Table 5]**

| PK Profile | ARSB | ARSB-Fc Fusion Protein | |
|---|---|---|---|
| Administration Conc. | 5.0 mg/kg (IV) | 5.0 mg/kg IV | 5.0 mg/kg (SC) |
| Degree of *in vivo* exposure (ng/mL^{∗}hr) | 30776.9 | 1230279.1 | 809176.0 |
| Maximum Drug Conc. in Blood (ng/mL) | 241588.2 | 166838.7 | 11679.2 |
| Blood Half-Life (hr) | Unable to calculate* | 74.2 | 33.4 |
| *In vivo* Bioavailability (%) | - | - | **65.8** |
| *Unable to calculate: T_{1/2} cannot be calculated due to extremely short half-life. | | | |

As can be seen in the above results, the long-acting fusion protein of arylsulfatase B according to the present invention showed significantly excellent pharmacokinetic characteristics compared to Naglazyme® (*i.e.,* naturally occurring arylsulfatase B). These results suggest that the long-acting fusion protein of arylsulfatase B of the present invention has the advantage of reducing the intervals of drug administration in the actual administration of the drug through the long-acting effect compared to enzymes.

As can be seen in the results of pharmacokinetics of FIG. 4 and Table 5, in the case of the long-acting fusion protein of arylsulfatase B, all of the half-life (T_{1/2}), maximum drug concentration in blood (Cₘₐₓ), and *in vivo* bioavailability (AUC) were increased. In particular, the *in vivo* bioavailability of the long-acting fusion protein of arylsulfatase B was 65.8%, thus showing excellent *in vivo* bioavailability compared to enzymes which are not long-acting fusion proteins.

As a result of the examination of the pharmacokinetics of enzyme fusion proteins in Examples 4-1 and 4-2, it was confirmed that these enzyme fusion proteins showed significantly increased half-lives, *in vivo* bioavailability, *etc.* compared to those enzymes to which an Fc region is not fused, and thus the long-acting effects of these enzyme fusion proteins can be expected.

### Example 5: Confirmation of enzyme activity of long-acting enzyme fusion protein

The activities of the enzymes included in the enzyme fusion proteins prepared above were compared to enzymes not fused with an Fc region.

### Example 5-1: In vitro enzyme activity of long-acting iduronate-2-sulfatase fusion protein

The present inventors made an attempt to measure the changes in enzyme activity according to the preparation of the long-acting fusion protein of iduronate-2-sulfatase prepared in Examples above, and as such, *in vitro* enzyme activity was measured.

Specifically, 4-methylumbelliferyl alpha-L-idopyranosiduronic acid-2-sulfate sodium salt (4MU-α-IdopyraA-2), which is known as an enzyme substrate, was reacted with iduronate-2-sulfatase and the long-acting fusion protein of iduronate-2-sulfatase at 37°C for 4 hours, and then reacted with *alpha*-iduronidase (*i.e.,* a secondary reaction enzyme) at 37°C for 24 hours. Then, the fluorescence of the final product, 4-methylumbelliferone (4MU), was measured to measure the enzyme activity for the corresponding material.

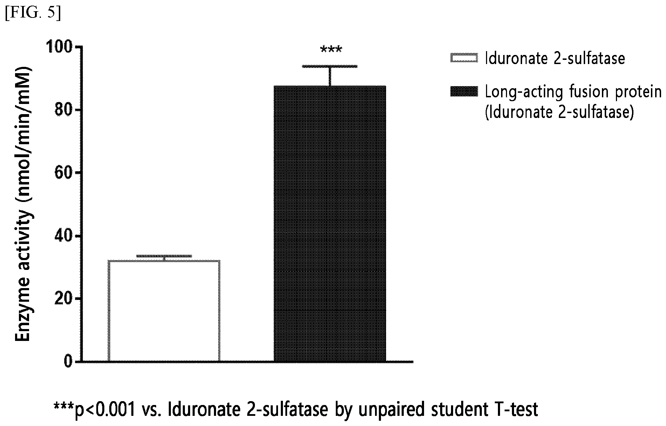

As a result, it was confirmed that iduronate-2-sulfatase and the long-acting fusion protein of iduronate-2-sulfatase had an enzyme activity (specific activity) of 32.0 ± 1.58 nmol/min/mM and 87.3 ± 6.49 nmol/min/mM, respectively. Since the long-acting fusion protein of iduronate-2-sulfatase has a structure in which two iduronate-2-sulfatase are linked to one Fc molecule, which is a dimeric form of two Fc chains, the measurement result showed that the long-acting fusion protein of iduronate-2-sulfatase has about 2.7-fold higher *in vitro* enzyme activity compared to iduronate-2-sulfatase, which is not a fusion protein. These results suggest that the structural characteristic of long-acting fusion protein of iduronate-2-sulfatase, which has two iduronate-2-sulfatase, has an advantage in the aspect of enzyme activity over the iduronate-2-sulfatase, which does not form a fusion protein (FIG. 5).

### Example 5-2: In vitro enzyme activity of long-acting arylsulfatase B fusion protein

The present inventors compared and measured the enzyme activity of the long-acting fusion protein of arylsulfatase B prepared in Examples above with that of the naturally occurring enzyme, arylsulfatase B (Naglazyme®, BioMarin).

Specifically, the long-acting fusion protein of arylsulfatase B and arylsulfatase B were reacted with 4-methylumbelliferyl sulfate at 37°C for 20 minutes, and the *in vitro* enzyme activity of the long-acting fusion protein of arylsulfatase B was measured by measuring the fluorescence of the 4-methylumbelliferyl formed after a sulfate group was cleaved.

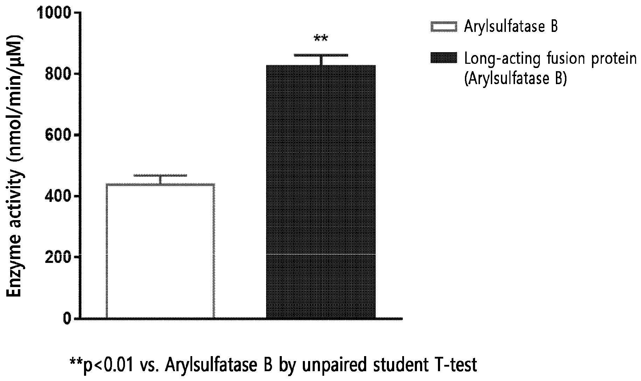

As a result, it was confirmed that it was confirmed that arylsulfatase B and the long-acting fusion protein of arylsulfatase B had an enzyme activity (specific activity) of 438.5 ± 29.4 nmol/min/mM and 823.8 ± 37.0 nmol/min/µM, respectively. Since the long-acting fusion protein of arylsulfatase B has a structure in which two arylsulfatase B are linked to one Fc molecule, which is a dimeric form of two Fc chains, the measurement result showed that the long-acting fusion protein of arylsulfatase B has about 1.9-fold higher *in vitro* enzyme activity compared to arylsulfatase B, which is not a fusion protein. These results confirmed that the distinguished structural characteristic of the long-acting fusion protein of arylsulfatase B at the molecular level over that of arylsulfatase B is ascribed to the excellent enzyme activity (FIG. 6).

As a result of the examination of the pharmacokinetics of enzyme fusion proteins in Examples 5-1 and 5-2, it was confirmed that these enzyme fusion proteins showed high *in vitro* enzyme activity compared to those enzymes to which an Fc region is not fused.

### Example 6: Confirmation of administration efficacy of long-acting iduronate-2-sulfatase fusion protein

The administration efficacy of the enzyme fusion protein of the present invention was examined by studying the changes in the amount of glycosaminoglycan (GAG) in the tissues and urine after administration of drugs to iduronate-2-sulfatase (IDS)-knockout mice.

Specifically, in addition to normal mice as the negative control group, 7- to 14-week-old IDS-knockout mice were divided into a total of four groups based on the GAG content in the urine. The iduronate-2-sulfatase (Elaprase®, Genzyme) was administered a total of 4 times (day 0, day 7, day 14, and day 21) to the caudal vein at a concentration of 0.5 mg/kg (control group). The administration of the long-acting fusion protein of iduronate-2-sulfatase proceeded by dividing into two groups: one group was administered once with the long-acting fusion protein of iduronate-2-sulfatase to the caudal vein at a concentration of 2.0 mg/kg (day 0), and the other group was subcutaneously administered once with the long-acting fusion protein of iduronate-2-sulfatase at a concentration of 4.0 mg/kg (day 0).

The urine samples were collected from each group before the administration, and on days 7, 14, 21, and 28 after the drug administration. All of the tissues from the liver, spleen, heart, and bone marrow were collected on day 28 after the drug administration. Then, the tissue pulverizing buffer (PBS containing aprotinin (1 µg/mL), 1 mM PMSF, and 2 mM EDTA) was added to each tissue in an amount of 5 volumes (9 volumes for bone marrow), and the mixture was pulverized using a sonicator and centrifuged, and each supernatant was used for the analysis of GAG contents.

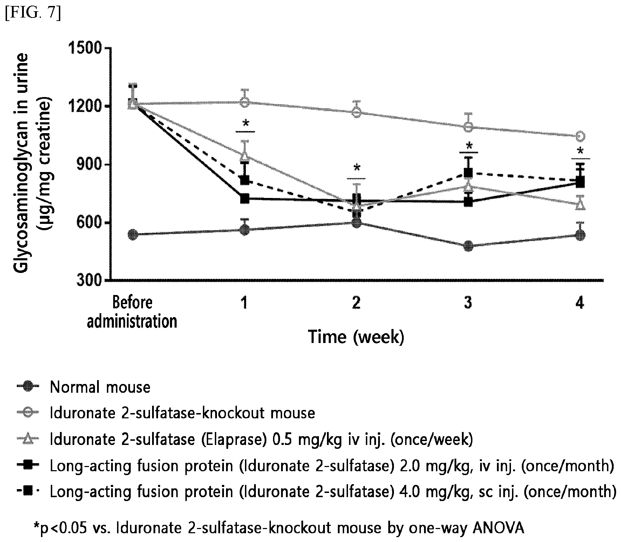

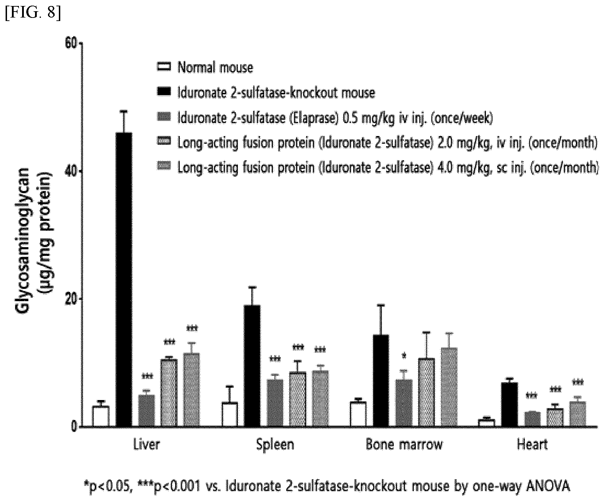

Then, 50 µL of the supernatant obtained after the pulverization of the collected urine samples and each tissue was added to a 96-well plate, and the dimethylmethylene blue solution (250 µL) was added and mixed, and the GAG contents were quantified at a wavelength of 525 nm. In the case of the GAG contents in the urine, the values were calculated by adjusting with reference to the amount of creatine. Statistical analysis was performed between the control and test groups using the one-way ANOVA using the calculated values. The measured GAG contents in the urine and each tissue are shown in FIGS. 7 and 8, respectively.

As shown in FIGS. 7 and 8, it was confirmed that the long-acting fusion protein of iduronate-2-sulfatase, even with a single intravenous or subcutaneous administration per month, significantly reduced the GAG values in the urine and each tissue to a level to similar to the a therapy where Elaprase®, which is an enzyme to which an Fc region is not fused, is intravenously administered once a week, compared to the IDS-knockout mice.

Through this Example, it was confirmed that due to the extended blood half-life, the long-acting fusion protein of iduronate-2-sulfatase, even with a single intravenous or subcutaneous administration per month, can exhibit an effect equivalent to the existing drug therapy where the drug is administered once a week. Additionally, the results that the long-acting fusion protein of iduronate-2-sulfatase exhibited an effect of reducing the GAG values in the group where the long-acting fusion protein of iduronate-2-sulfatase was subcutaneously administered once a month, confirmed that the long-acting fusion protein of iduronate-2-sulfatase has the potential to be used for the subcutaneous injection as an administration route of the fusion proteins of the present invention. Accordingly, it is suggested that the long-acting fusion protein of iduronate-2-sulfatase according to the present invention may be used to treat Hunter syndrome patients through the administration or subcutaneous administration once per month.

### Example 7: Confirmation of tissue distribution of long-acting fusion protein (arylsulfatase B)

The present inventors made an attempt to confirm the degree of distribution of the enzyme fusion proteins of the present invention prepared in Examples above.

In this regard, the degree of distribution of the arylsulfatase B (control group) and the long-acting fusion protein of arylsulfatase B (experimental group) in tissues and organs of 3 ICR mice were compared for each sample collection and according to each group.

Specifically, the control group and the experimental group were administered by intravenous injection at a concentration of 5.0 mg/kg, based on the concentration of arylsulfatase B.

With regard to the naturally occurring arylsulfatase B (Naglazyme®) of the control group and the long-acting fusion protein of arylsulfatase B of the experimental group, the concentration of each material in tissues (bone marrow, livers, spleen, lungs, kidneys, and hearts) was measured and compared by the enzyme immunoassay after the organs of the mice were removed following the administration of the naturally occurring arylsulfatase B (Naglazyme®) and the long-acting fusion protein of arylsulfatase B.

As a result, the long-acting fusion protein of arylsulfatase B showed a result that it is distributed in a higher degree or for a longer period of time at the same time section in all of the tissues, compared to the naturally occurring arylsulfatase B, which was used as the control group and to which an Fc region is not fused.

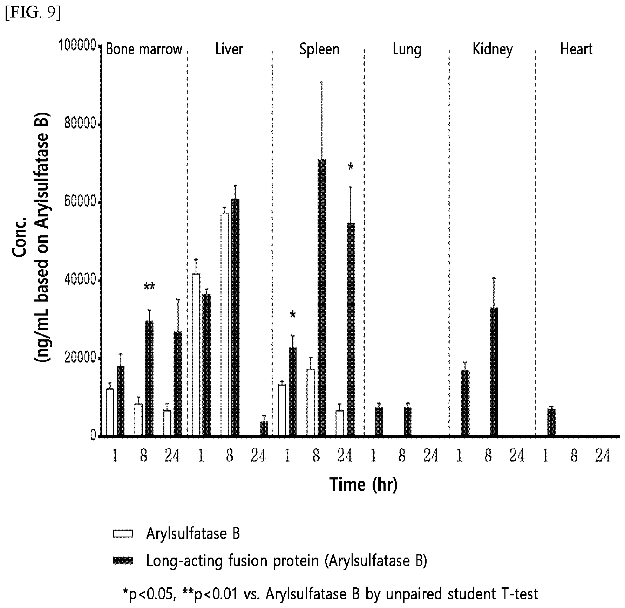

In particular, it was confirmed that the long-acting fusion protein of arylsulfatase B has a significantly high degree of distribution in the bone marrow and spleen, compared to the naturally occurring arylsulfatase B. Additionally, it was confirmed that the long-acting fusion protein of arylsulfatase B was distributed in the lungs, kidneys, and hearts while arylsulfatase B was not detected in those tissues (FIG. 9).

From these experimental results, it was confirmed that the long-acting fusion protein of arylsulfatase B has excellent pharmacokinetic characteristics over Naglazyme® (*i.e.,* a naturally occurring arylsulfatase B). In particular, the potential use of the long-acting fusion protein of arylsulfatase B for once-per-month administration, compared to the existing once-per-week intravenous administration therapy, can not only reduce the administration frequency but can also contribute to the improvement of patients' quality of life through the conversion into the subcutaneous administration.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. An enzyme fusion protein, wherein an immunoglobulin Fc region is fused to a therapeutic enzyme and the therapeutic enzyme has increased *in vivo* duration compared to a therapeutic enzyme to which an immunoglobulin Fc region is not fused.

2. The enzyme fusion protein of claim 1, wherein the therapeutic enzyme is selected from the group consisting of *beta*-glucosidase, *alpha*-galactosidase, *beta*-galactosidase, iduronidase, iduronate-2-sulfatase, galactose-6-sulfatase, acid *alpha*-glucosidase, acid ceramidase, acid sphingomyelinsase, galactocerebrosidsase, arylsulfatase A, B, *beta*-hexosaminidase A, B, heparin N-sulfatase, *alpha*-D-mannosidase, *beta*-glucuronidase, N-acetylgalactosamine-6 sulfatase, lysosomal acid lipase, *alpha*-N-acetyl-glucosaminidase, glucocerebrosidase, butyrylcholinesterase, chitinase, glutamate decarboxylase, imiglucerase, lipase, uricase, platelet-activating factor acetylhydrolase, neutral endopeptidase, and myeloperoxidase.

3. The enzyme fusion protein of claim 1, wherein a therapeutic enzyme and an immunoglobulin Fc region are fused by a peptide linker.

4. The enzyme fusion protein of claim 1, wherein the enzyme fusion protein is a fusion of one molecule of immunoglobulin Fc region and a dimeric therapeutic enzyme.

5. The enzyme fusion protein of claim 1, wherein the immunoglobulin Fc region has a variation selected from the group consisting of substitution, addition, deletion, modification, and a combination thereof in at least one amino acid of a native immunoglobulin Fc region.

6. The enzyme fusion protein of claim 5, wherein, in the immunoglobulin Fc region having the amino sequence of SEQ ID NO: 8, the 2^{nd} amino acid is substituted with proline; the 71^{st} amino acid is substituted with glutamine; or the 2^{nd} amino acid is substituted with proline and the 71^{st} amino acid is substituted with glutamine.

7. The enzyme fusion protein of claim 6, wherein no chain exchange occurs in the immunoglobulin Fc region.

8. The enzyme fusion protein of claim 1, wherein the enzyme fusion protein has increased stability and reduced binding affinity for lysosome receptors, thereby having a high degree of tissue distribution, compared to a therapeutic enzyme therapeutic enzyme to which an immunoglobulin Fc region is not fused.

9. The enzyme fusion protein of claim 1, wherein the immunoglobulin Fc region is selected from the group consisting of
(a) a CH1 domain, a CH2 domain, a CH3 domain, and a CH4 domain;
(b) a CH1 domain and a CH2 domain;
(c) a CH1 domain and a CH3 domain;
(d) a CH2 domain and a CH3 domain;
(e) a combination between one or two or more domains among a CH1 domain, a CH2 domain, a CH3 domain, and a CH4 domain and an immunoglobulin hinge region or a part of the hinge region; and
(f) a dimer between each domain of the heavy chain constant region and the light chain constant region.

10. The enzyme fusion protein of claim 1, wherein the immunoglobulin Fc region is selected from the group consisting of (a) a region capable of forming a disulfide bond is removed, (b) a certain amino acid residue is removed at the N-terminus of a native Fc, (c) a methionine residue is added at the N-terminus of a native Fc form, (d) a complement-binding site is removed, or (e) an antibody-dependent cell-mediated cytotoxicity (ADCC) site is deleted.

11. The enzyme fusion protein of any one of claims 1 to 10, wherein the immunoglobulin Fc region is aglycosylated.

12. The enzyme fusion protein of any one of claims 1 to 10, wherein the immunoglobulin Fc region is an Fc fragment derived from IgG, IgA, IgD, IgE, or IgM.

13. The enzyme fusion protein of claim 12, wherein the immunoglobulin Fc region is a hybrid of domains having different origins derived from immunoglobulins selected from the group consisting of IgG, IgA, IgD, IgE, and IgM.

14. The enzyme fusion protein of claim 13, wherein the immunoglobulin Fc region is an IgG4 Fc region.

15. The enzyme fusion protein of claim 14, wherein the hinge region of the IgG4 Fc region is substituted with an IgG1 hinge region.

16. A pharmaceutical composition for preventing or treating lysosomal storage disorder (LSD) comprising the enzyme fusion protein of any one of claims 1 to 10.

17. The pharmaceutical composition of claim 16, wherein the lysosomal storage disorder (LSD) is selected from the group consisting of mucopolysaccharidosis (MPS), glycogen storage disease, sphingolipidosis, Niemann-Pick disease, Fabry's disease, Gaucher disease, Hunter syndrome, and Maroteaux-Lamy syndrome.

18. The pharmaceutical composition of claim 16, wherein the enzyme is iduronate-2-sulfatase (IDS) or arylsulfatase B (ARSB).

19. The pharmaceutical composition of claim 16, wherein the composition reduces the binding affinity of an enzyme for lysosome receptors.

20. A polynucleotide encoding the enzyme fusion protein of any one of claims 1 to 10.

21. An expression vector comprising the polynucleotide of claim 20.

22. A transformant into which the expression vector of claim 21 is introduced.

23. A method for preparing an enzyme fusion protein, comprising:
(a) culturing the transformant of claim 22 to obtain a culture; and
(b) recovering an enzyme fusion protein from the culture.


<110>     HANMI PHARM. CO., LTD.

<120>     Novel therapeutic enzyme fusion protein and use thereof

<130>     OPA18228

<150>     KR 10-2017-0086594
<151>     2017-07-07

<160>     26

<170>     KoPatentIn 3.0

<210>     1
<211>     1650
<212>     DNA
<213>     Homo Sapiens

<400>     1
atgccgccac cccggaccgg ccgaggcctt ctctggctgg gtctggttct gagctccgtc          60

tgcgtcgccc tcggatccga aacgcaggcc aactcgacca cagatgctct gaacgttctt         120

ctcatcatcg tggatgacct gcgcccctcc ctgggctgtt atggggataa gctggtgagg         180

tccccaaata ttgaccaact ggcatcccac agcctcctct tccagaatgc ctttgcgcag         240

caagcagtgt gcgccccgag ccgcgtttct ttcctcactg gcaggagacc tgacaccacc         300

cgcctgtacg acttcaactc ctactggagg gtgcacgctg gaaacttctc caccatcccc         360

cagtacttca aggagaatgg ctatgtgacc atgtcggtgg gaaaagtctt tcaccctggg         420

atatcttcta accataccga tgattctccg tatagctggt cttttccacc ttatcatcct         480

tcctctgaga agtatgaaaa cactaagaca tgtcgagggc cagatggaga actccatgcc         540

aacctgcttt gccctgtgga tgtgctggat gttcccgagg gcaccttgcc tgacaaacag         600

agcactgagc aagccataca gttgttggaa aagatgaaaa cgtcagccag tcctttcttc         660

ctggccgttg ggtatcataa gccacacatc cccttcagat accccaagga atttcagaag         720

ttgtatccct tggagaacat caccctggcc cccgatcccg aggtccctga tggcctaccc         780

cctgtggcct acaacccctg gatggacatc aggcaacggg aagacgtcca agccttaaac         840

atcagtgtgc cgtatggtcc aattcctgtg gactttcagc ggaaaatccg ccagagctac         900

tttgcctctg tgtcatattt ggatacacag gtcggccgcc tcttgagtgc tttggacgat         960

cttcagctgg ccaacagcac catcattgca tttacctcgg atcatgggtg ggctctaggt        1020

gaacatggag aatgggccaa atacagcaat tttgatgttg ctacccatgt tcccctgata        1080

ttctatgttc ctggaaggac ggcttcactt ccggaggcag gcgagaagct tttcccttac        1140

ctcgaccctt ttgattccgc ctcacagttg atggagccag gcaggcaatc catggacctt        1200

gtggaacttg tgtctctttt tcccacgctg gctggacttg caggactgca ggttccacct        1260

cgctgccccg ttccttcatt tcacgttgag ctgtgcagag aaggcaagaa ccttctgaag        1320

cattttcgat tccgtgactt ggaagaggat ccgtacctcc ctggtaatcc ccgtgaactg 1380

attgcctata gccagtatcc ccggccttca gacatccctc agtggaattc tgacaagccg 1440

agtttaaaag atataaagat catgggctat tccatacgca ccatagacta taggtatact 1500

gtgtgggttg gcttcaatcc tgatgaattt ctagctaact tttctgacat ccatgcaggg 1560

gaactgtatt ttgtggattc tgacccattg caggatcaca atatgtataa tgattcccaa 1620

ggtggagatc ttttccagtt gttgatgcct 1650

<210> 2
<211> 550
<212> PRT
<213> Homo sapiens

<400> 2
Met Pro Pro Pro Arg Thr Gly Arg Gly Leu Leu Trp Leu Gly Leu Val
1               5                   10                  15

Leu Ser Ser Val Cys Val Ala Leu Gly Ser Glu Thr Gln Ala Asn Ser
            20                  25                  30

Thr Thr Asp Ala Leu Asn Val Leu Leu Ile Ile Val Asp Asp Leu Arg
        35                  40                  45

Pro Ser Leu Gly Cys Tyr Gly Asp Lys Leu Val Arg Ser Pro Asn Ile
    50                  55                  60

Asp Gln Leu Ala Ser His Ser Leu Leu Phe Gln Asn Ala Phe Ala Gln
65                  70                  75                  80

Gln Ala Val Cys Ala Pro Ser Arg Val Ser Phe Leu Thr Gly Arg Arg
                85                  90                  95

Pro Asp Thr Thr Arg Leu Tyr Asp Phe Asn Ser Tyr Trp Arg Val His
            100                 105                 110

Ala Gly Asn Phe Ser Thr Ile Pro Gln Tyr Phe Lys Glu Asn Gly Tyr
        115                 120                 125

Val Thr Met Ser Val Gly Lys Val Phe His Pro Gly Ile Ser Ser Asn
    130                 135                 140

His Thr Asp Asp Ser Pro Tyr Ser Trp Ser Phe Pro Pro Tyr His Pro
145                 150                 155                 160

Ser Ser Glu Lys Tyr Glu Asn Thr Lys Thr Cys Arg Gly Pro Asp Gly
                165                 170                 175

Glu Leu His Ala Asn Leu Leu Cys Pro Val Asp Val Leu Asp Val Pro
            180                 185                 190

Glu Gly Thr Leu Pro Asp Lys Gln Ser Thr Glu Gln Ala Ile Gln Leu
        195                 200                 205

Leu Glu Lys Met Lys Thr Ser Ala Ser Pro Phe Phe Leu Ala Val Gly
    210                 215                 220

Tyr His Lys Pro His Ile Pro Phe Arg Tyr Pro Lys Glu Phe Gln Lys
225                 230                 235                 240

Leu Tyr Pro Leu Glu Asn Ile Thr Leu Ala Pro Asp Pro Glu Val Pro
                245                 250                 255

Asp Gly Leu Pro Pro Val Ala Tyr Asn Pro Trp Met Asp Ile Arg Gln
            260                 265                 270

Arg Glu Asp Val Gln Ala Leu Asn Ile Ser Val Pro Tyr Gly Pro Ile
        275                 280                 285

Pro Val Asp Phe Gln Arg Lys Ile Arg Gln Ser Tyr Phe Ala Ser Val
    290                 295                 300

Ser Tyr Leu Asp Thr Gln Val Gly Arg Leu Leu Ser Ala Leu Asp Asp
305                 310                 315                 320

Leu Gln Leu Ala Asn Ser Thr Ile Ile Ala Phe Thr Ser Asp His Gly
                325                 330                 335

Trp Ala Leu Gly Glu His Gly Glu Trp Ala Lys Tyr Ser Asn Phe Asp
            340                 345                 350

Val Ala Thr His Val Pro Leu Ile Phe Tyr Val Pro Gly Arg Thr Ala
        355                 360                 365

Ser Leu Pro Glu Ala Gly Glu Lys Leu Phe Pro Tyr Leu Asp Pro Phe
    370                 375                 380

Asp Ser Ala Ser Gln Leu Met Glu Pro Gly Arg Gln Ser Met Asp Leu
385                 390                 395                 400

Val Glu Leu Val Ser Leu Phe Pro Thr Leu Ala Gly Leu Ala Gly Leu
                405                 410                 415

Gln Val Pro Pro Arg Cys Pro Val Pro Ser Phe His Val Glu Leu Cys
            420                 425                 430

Arg Glu Gly Lys Asn Leu Leu Lys His Phe Arg Phe Arg Asp Leu Glu
        435                 440                 445

Glu Asp Pro Tyr Leu Pro Gly Asn Pro Arg Glu Leu Ile Ala Tyr Ser
    450                 455                 460

Gln Tyr Pro Arg Pro Ser Asp Ile Pro Gln Trp Asn Ser Asp Lys Pro
465                 470                 475                 480

Ser Leu Lys Asp Ile Lys Ile Met Gly Tyr Ser Ile Arg Thr Ile Asp
                485                 490                 495

Tyr Arg Tyr Thr Val Trp Val Gly Phe Asn Pro Asp Glu Phe Leu Ala
            500                 505                 510

Asn Phe Ser Asp Ile His Ala Gly Glu Leu Tyr Phe Val Asp Ser Asp
        515                 520                 525

Pro Leu Gln Asp His Asn Met Tyr Asn Asp Ser Gln Gly Gly Asp Leu
    530                 535                 540

Phe Gln Leu Leu Met Pro
545                 550

<210>   3

<211>    1599
<212>    DNA
<213>    Homo Sapiens

<400>    3
atgggtccgc gcggcgcggc gagcttgccc cgaggccccg gtcctcggcg gctgcttctc      60

cccgtcgtcc tcccgctgct gctgctgctg ttgttggcgc cgccgggctc gggcgccggg     120

gccagccggc cgccccacct ggtcttcttg ctggcagacg acctaggctg gaacgacgtc     180

ggcttccacg gctcccgcat ccgcacgccg cacctggacg cgctggcggc cggcggggtg     240

ctcctggaca actactacac gcagccgctg tgcacgccgt cgcggagcca gctgctcact     300

ggccgctacc agatccgtac aggtttacag caccaaataa tctggccctg tcagcccagc     360

tgtgttcctc tggatgaaaa actcctgccc cagctcctaa aagaagcagg ttatactacc     420

catatggtcg gaaaatggca cctgggaatg taccggaaag aatgccttcc aacccgccga     480

ggatttgata cctactttgg atatctcctg ggtagtgaag attattattc ccatgaacgc     540

tgtacattaa ttgacgctct gaatgtcaca cgatgtgctc ttgattttcg agatggcgaa     600

gaagttgcaa caggatataa aaatatgtat tcaacaaaca tattcaccaa aagggctata     660

gccctcataa ctaaccatcc accagagaag cctctgtttc tctaccttgc tctccagtct     720

gtgcatgagc cccttcaggt ccctgaggaa tacttgaagc catatgactt tatccaagac     780

aagaacaggc atcactatgc aggaatggtg tcccttatgg atgaagcagt aggaaatgtc     840

actgcagctt taaaaagcag tgggctctgg aacaacacgg tgttcatctt ttctacagat     900

aacggagggc agactttggc agggggtaat aactggcccc ttcgaggaag aaaatggagc     960

ctgtgggaag gaggcgtccg aggggtgggc tttgtggcaa gccccttgct gaagcagaag    1020

ggcgtgaaga accgggagct catccacatc tctgactggc tgccaacact cgtgaagctg    1080

gccaggggac acaccaatgg cacaaagcct ctggatggct tcgacgtgtg gaaaaccatc    1140

agtgaaggaa gcccatcccc cagaattgag ctactgcata atattgaccc gaacttcgtg    1200

gactcttcac cgtgtcccag gaacagcatg gctccagcaa aggatgactc ttctcttcca    1260

gaatattcag cctttaacac atctgtccat gctgcaatta gacatggaaa ttggaaactc    1320

ctcacgggct acccaggctg tggttactgg ttccctccac cgtctcaata caatgtttct    1380

gagataccct catcagaccc accaaccaag accctctggc tctttgatat tgatcgggac    1440

cctgaagaaa gacatgacct gtccagagaa tatcctcaca tcgtcacaaa gctcctgtcc    1500

cgcctacagt tctaccataa acactcagtc cccgtgtact tccctgcaca ggacccccgc    1560

tgtgatccca aggccactgg ggtgtggggc ccttggatg                           1599

<210>    4
<211>    533
<212>    PRT

<213>    Homo Sapiens

<400>    4


<210>    5
<211>    45
<212>    DNA
<213>    Artificial Sequence

<220>
<223>    Peptide Linker


<400>    5
ggtggaggcg gttcaggcgg aggtggctct ggcggtggcg gatcg                    45


<210>    6
<211>    15
<212>    PRT
<213>    Artificial Sequence

<220>
<223>    Peptide Linker

<400>    6
Gly Gly Gly Gly Ser Gly Gly Gly Gly Ser Gly Gly Gly Gly Ser
1               5                   10                  15

<210>    7
<211>    666
<212>    DNA
<213>    Artificial Sequence

<220>
<223>    Immunoglobulin Fc variant

<400>    7
ccatcatgcc cagcacctga gttcctgggg ggaccatcag tcttcctgtt ccccccaaaa      60

cccaaggaca ccctcatgat ctcccggacc cctgaggtca catgcgtggt ggtggacgtg     120

agccaggaag accctgaggt ccagttcaac tggtacgtgg acggcgtgga ggtgcataat     180

gccaagacaa agccgcggga ggagcagttc aacagcacgt accgtgtggt cagcgtcctc     240

accgtcctgc accaggactg gctgaatggc aaggagtaca agtgcaaggt ctccaacaaa     300

ggcctcccat cctccatcga gaaaaccatc tccaaagcca aagggcagcc ccgagaacca     360

caggtgtaca ccctgccccc atcccaggag gagatgacca agaaccaggt cagcctgacc     420

tgcctggtca aaggcttcta tcccagcgac atcgccgtgg agtgggagag caatgggcag     480

ccggagaaca actacaagac cacgcctccc gtgctggact ccgacggctc cttcttcctc     540

tacagcaggc taaccgtgga caagagcagg tggcaggagg ggaacgtctt ctcatgctcc     600

gtgatgcatg aggctctgca caaccactac acgcagaaga gcctctccct gtctctgggt     660

aaatga                                                                666

<210>    8
<211>    221
<212>    PRT
<213>    Artificial Sequence

<220>
<223>    IgG4 Fc

<400>    8
Pro Ser Cys Pro Ala Pro Glu Phe Leu Gly Gly Pro Ser Val Phe Leu
1               5                   10                  15

Phe Pro Pro Lys Pro Lys Asp Thr Leu Met Ile Ser Arg Thr Pro Glu
            20                  25                  30

Val Thr Cys Val Val Val Asp Val Ser Gln Glu Asp Pro Glu Val Gln
        35                  40                  45

Phe Asn Trp Tyr Val Asp Gly Val Glu Val His Asn Ala Lys Thr Lys
    50                  55                  60

Pro Arg Glu Glu Gln Phe Asn Ser Thr Tyr Arg Val Val Ser Val Leu
65                  70                  75                  80

Thr Val Leu His Gln Asp Trp Leu Asn Gly Lys Glu Tyr Lys Cys Lys
                85                  90                  95

Val Ser Asn Lys Gly Leu Pro Ser Ser Ile Glu Lys Thr Ile Ser Lys
            100                 105                 110

Ala Lys Gly Gln Pro Arg Glu Pro Gln Val Tyr Thr Leu Pro Pro Ser
        115                 120                 125

Gln Glu Glu Met Thr Lys Asn Gln Val Ser Leu Thr Cys Leu Val Lys
    130                 135                 140

Gly Phe Tyr Pro Ser Asp Ile Ala Val Glu Trp Glu Ser Asn Gly Gln
145                 150                 155                 160

Pro Glu Asn Asn Tyr Lys Thr Thr Pro Pro Val Leu Asp Ser Asp Gly
                165                 170                 175

Ser Phe Phe Leu Tyr Ser Arg Leu Thr Val Asp Lys Ser Arg Trp Gln
            180                 185                 190

Glu Gly Asn Val Phe Ser Cys Ser Val Met His Glu Ala Leu His Asn
        195                 200                 205

His Tyr Thr Gln Lys Ser Leu Ser Leu Ser Leu Gly Lys
    210                 215                 220

<210>   9
<211>   221
<212>   PRT
<213>   Artificial Sequence

<220>
<223>   Immunoglobulin Fc variant

<400>   9
Pro Pro Cys Pro Ala Pro Glu Phe Leu Gly Gly Pro Ser Val Phe Leu
 1               5                  10                  15

Phe Pro Pro Lys Pro Lys Asp Thr Leu Met Ile Ser Arg Thr Pro Glu
            20                  25                  30

Val Thr Cys Val Val Val Asp Val Ser Gln Glu Asp Pro Glu Val Gln
        35                  40                  45

Phe Asn Trp Tyr Val Asp Gly Val Glu Val His Asn Ala Lys Thr Lys
    50                  55                  60

Pro Arg Glu Glu Gln Phe Gln Ser Thr Tyr Arg Val Val Ser Val Leu
65                  70                  75                  80

Thr Val Leu His Gln Asp Trp Leu Asn Gly Lys Glu Tyr Lys Cys Lys
                85                  90                  95

Val Ser Asn Lys Gly Leu Pro Ser Ser Ile Glu Lys Thr Ile Ser Lys
            100                 105                 110

Ala Lys Gly Gln Pro Arg Glu Pro Gln Val Tyr Thr Leu Pro Pro Ser
        115                 120                 125

Gln Glu Glu Met Thr Lys Asn Gln Val Ser Leu Thr Cys Leu Val Lys
    130                 135                 140

Gly Phe Tyr Pro Ser Asp Ile Ala Val Glu Trp Glu Ser Asn Gly Gln
145                 150                 155                 160

Pro Glu Asn Asn Tyr Lys Thr Thr Pro Pro Val Leu Asp Ser Asp Gly
                165                 170                 175

Ser Phe Phe Leu Tyr Ser Arg Leu Thr Val Asp Lys Ser Arg Trp Gln
            180                 185                 190

Glu Gly Asn Val Phe Ser Cys Ser Val Met His Glu Ala Leu His Asn
        195                 200                 205

His Tyr Thr Gln Lys Ser Leu Ser Leu Ser Leu Gly Lys
    210                 215                 220

<210>    10
<211>    26
<212>    DNA
<213>    Artificial Sequence

<220>
<223>    IDS-F (KpnI)

<400>    10
caggtaccat gccgccaccc cggacc                                          26

<210>    11
<211>    44
<212>    DNA
<213>    Artificial Sequence

<220>
<223>    IDS-R (overlap)

<400>    11
tgaaccgcct ccaccaggca tcaacaactg gaaaagatct ccac                      44

<210>    12
<211>    34
<212>    DNA
<213>    Artificial Sequence

<220>
<223>    L15Fc(IDS)-F

<400>    12
cagttgttga tgcctggtgg aggcggttca ggcg                                 34

<210>	13
<211>	33
<212>	DNA
<213>	Artificial Sequence

<220>
<223>	L15Fc-R (XhoI)


<400>	13
gactcgagtc atttacccag agacagggag agg	33


<210>	14
<211>	26
<212>	DNA
<213>	Artificial Sequence

<220>
<223>	ARSB-F (KpnI)


<400>	14
caggtaccat gggtccgcgc ggcgcg	26


<210>	15
<211>	35
<212>	DNA
<213>	Artificial Sequence

<220>
<223>	ARSB-R (overlap)


<400>	15
tgaaccgcct ccacccatcc aagggcccca caccc	35


<210>	16
<211>	34
<212>	DNA
<213>	Artificial Sequence

<220>
<223>	L15Fc(ARSB)-F


<400>	16
tggggccctt ggatgggtgg aggcggttca ggcg	34


<210>	17
<211>	33
<212>	DNA
<213>	Artificial Sequence

<220>
<223>	L15Fc-R (XhoI)


<400>	17
gactcgagtc atttacccag agacagggag agg	33

<210>	18
<211>	43
<212>	DNA
<213>	Artificial Sequence

<220>
<223>	Fc(S2P)_F


<400>	18
ctggcggtgg cggatcgcca ccatgcccag cacctgagtt cct	43


<210>	19
<211>	43
<212>	DNA
<213>	Artificial Sequence

<220>
<223>	Fc(S2P)_R


<400>	19
aggaactcag gtgctgggca tggtggcgat ccgccaccgc cag	43


<210>	20
<211>	43
<212>	DNA
<213>	Artificial Sequence

<220>
<223>	Fc(N71Q)_F


<400>	20
agccgcggga ggagcagttc caaagcacgt accgtgtggt cag	43


<210>	21
<211>	43
<212>	DNA
<213>	Artificial Sequence

<220>
<223>	Fc(N71Q)_R


<400>	21
ctgaccacac ggtacgtgct ttggaactgc tcctcccgcg gct	43


<210>	22
<211>	2361
<212>	DNA
<213>	Artificial Sequence

<220>
<223>	IDS-Fc

<400>    22
atgccgccac cccggaccgg ccgaggcctt ctctggctgg gtctggttct gagctccgtc      60

tgcgtcgccc tcggatccga aacgcaggcc aactcgacca cagatgctct gaacgttctt     120

ctcatcatcg tggatgacct gcgcccctcc ctgggctgtt atggggataa gctggtgagg     180

tccccaaata ttgaccaact ggcatcccac agcctcctct tccagaatgc ctttgcgcag     240

caagcagtgt gcgccccgag ccgcgtttct ttcctcactg gcaggagacc tgacaccacc     300

cgcctgtacg acttcaactc ctactggagg gtgcacgctg gaaacttctc caccatcccc     360

cagtacttca aggagaatgg ctatgtgacc atgtcggtgg gaaaagtctt tcaccctggg     420

atatcttcta accataccga tgattctccg tatagctggt cttttccacc ttatcatcct     480

tcctctgaga agtatgaaaa cactaagaca tgtcgagggc cagatggaga actccatgcc     540

aacctgcttt gccctgtgga tgtgctggat gttcccgagg gcaccttgcc tgacaaacag     600

agcactgagc aagccataca gttgttggaa aagatgaaaa cgtcagccag tcctttcttc     660

ctggccgttg ggtatcataa gccacacatc cccttcagat accccaagga atttcagaag     720

ttgtatccct tggagaacat caccctggcc cccgatcccg aggtccctga tggcctaccc     780

cctgtggcct acaacccctg gatggacatc aggcaacggg aagacgtcca agccttaaac     840

atcagtgtgc cgtatggtcc aattcctgtg gactttcagc ggaaaatccg ccagagctac     900

tttgcctctg tgtcatattt ggatacacag gtcggccgcc tcttgagtgc tttggacgat     960

cttcagctgg ccaacagcac catcattgca tttacctcgg atcatgggtg ggctctaggt    1020

gaacatggag aatgggccaa atacagcaat tttgatgttg ctacccatgt tcccctgata    1080

ttctatgttc ctggaaggac ggcttcactt ccggaggcag gcgagaagct tttcccttac    1140

ctcgaccctt ttgattccgc ctcacagttg atggagccag gcaggcaatc catggacctt    1200

gtggaacttg tgtctctttt tcccacgctg gctggacttg caggactgca ggttccacct    1260

cgctgccccg ttccttcatt tcacgttgag ctgtgcagag aaggcaagaa ccttctgaag    1320

cattttcgat tccgtgactt ggaagaggat ccgtacctcc ctggtaatcc ccgtgaactg    1380

attgcctata gccagtatcc ccggccttca gacatccctc agtggaattc tgacaagccg    1440

agtttaaaag atataaagat catgggctat tccatacgca ccatagacta taggtatact    1500

gtgtgggttg gcttcaatcc tgatgaattt ctagctaact tttctgacat ccatgcaggg    1560

gaactgtatt ttgtggattc tgacccattg caggatcaca atatgtataa tgattcccaa    1620

ggtggagatc ttttccagtt gttgatgcct ggtggaggcg gttcaggcgg aggtggctct    1680

ggcggtggcg gatcgccatc atgcccagca cctgagttcc tggggggacc atcagtcttc    1740

ctgttccccc caaaacccaa ggacaccctc atgatctccc ggacccctga ggtcacatgc    1800

gtggtggtgg acgtgagcca ggaagaccct gaggtccagt tcaactggta cgtggacggc    1860

gtggaggtgc ataatgccaa gacaaagccg cgggaggagc agttcaacag cacgtaccgt 1920

gtggtcagcg tcctcaccgt cctgcaccag gactggctga atggcaagga gtacaagtgc 1980

aaggtctcca acaaaggcct cccatcctcc atcgagaaaa ccatctccaa agccaaaggg 2040

cagccccgag aaccacaggt gtacaccctg cccccatccc aggaggagat gaccaagaac 2100

caggtcagcc tgacctgcct ggtcaaaggc ttctatccca gcgacatcgc cgtggagtgg 2160

gagagcaatg ggcagccgga gaacaactac aagaccacgc ctcccgtgct ggactccgac 2220

ggctccttct tcctctacag caggctaacc gtggacaaga gcaggtggca ggaggggaac 2280

gtcttctcat gctccgtgat gcatgaggct ctgcacaacc actacacgca gaagagcctc 2340

tccctgtctc tgggtaaatg a 2361

<210> 23
<211> 786
<212> PRT
<213> Artificial Sequence

<220>
<223> IDS-Fc

<400> 23
Met Pro Pro Pro Arg Thr Gly Arg Gly Leu Leu Trp Leu Gly Leu Val
1               5                   10                  15

Leu Ser Ser Val Cys Val Ala Leu Gly Ser Glu Thr Gln Ala Asn Ser
            20                  25                  30

Thr Thr Asp Ala Leu Asn Val Leu Leu Ile Ile Val Asp Asp Leu Arg
        35                  40                  45

Pro Ser Leu Gly Cys Tyr Gly Asp Lys Leu Val Arg Ser Pro Asn Ile
    50                  55                  60

Asp Gln Leu Ala Ser His Ser Leu Leu Phe Gln Asn Ala Phe Ala Gln
65                  70                  75                  80

Gln Ala Val Cys Ala Pro Ser Arg Val Ser Phe Leu Thr Gly Arg Arg
                85                  90                  95

Pro Asp Thr Thr Arg Leu Tyr Asp Phe Asn Ser Tyr Trp Arg Val His
            100                 105                 110

Ala Gly Asn Phe Ser Thr Ile Pro Gln Tyr Phe Lys Glu Asn Gly Tyr
        115                 120                 125

Val Thr Met Ser Val Gly Lys Val Phe His Pro Gly Ile Ser Ser Asn
    130                 135                 140

His Thr Asp Asp Ser Pro Tyr Ser Trp Ser Phe Pro Pro Tyr His Pro
145                 150                 155                 160

Ser Ser Glu Lys Tyr Glu Asn Thr Lys Thr Cys Arg Gly Pro Asp Gly
                165                 170                 175

Glu Leu His Ala Asn Leu Leu Cys Pro Val Asp Val Leu Asp Val Pro

180                 185                 190

Glu Gly Thr Leu Pro Asp Lys Gln Ser Thr Glu Gln Ala Ile Gln Leu
        195                 200                 205

Leu Glu Lys Met Lys Thr Ser Ala Ser Pro Phe Phe Leu Ala Val Gly
    210                 215                 220

Tyr His Lys Pro His Ile Pro Phe Arg Tyr Pro Lys Glu Phe Gln Lys
225                 230                 235                 240

Leu Tyr Pro Leu Glu Asn Ile Thr Leu Ala Pro Asp Pro Glu Val Pro
                245                 250                 255

Asp Gly Leu Pro Pro Val Ala Tyr Asn Pro Trp Met Asp Ile Arg Gln
            260                 265                 270

Arg Glu Asp Val Gln Ala Leu Asn Ile Ser Val Pro Tyr Gly Pro Ile
        275                 280                 285

Pro Val Asp Phe Gln Arg Lys Ile Arg Gln Ser Tyr Phe Ala Ser Val
    290                 295                 300

Ser Tyr Leu Asp Thr Gln Val Gly Arg Leu Leu Ser Ala Leu Asp Asp
305                 310                 315                 320

Leu Gln Leu Ala Asn Ser Thr Ile Ile Ala Phe Thr Ser Asp His Gly
                325                 330                 335

Trp Ala Leu Gly Glu His Gly Glu Trp Ala Lys Tyr Ser Asn Phe Asp
            340                 345                 350

Val Ala Thr His Val Pro Leu Ile Phe Tyr Val Pro Gly Arg Thr Ala
        355                 360                 365

Ser Leu Pro Glu Ala Gly Glu Lys Leu Phe Pro Tyr Leu Asp Pro Phe
    370                 375                 380

Asp Ser Ala Ser Gln Leu Met Glu Pro Gly Arg Gln Ser Met Asp Leu
385                 390                 395                 400

Val Glu Leu Val Ser Leu Phe Pro Thr Leu Ala Gly Leu Ala Gly Leu
                405                 410                 415

Gln Val Pro Pro Arg Cys Pro Val Pro Ser Phe His Val Glu Leu Cys
            420                 425                 430

Arg Glu Gly Lys Asn Leu Leu Lys His Phe Arg Phe Arg Asp Leu Glu
        435                 440                 445

Glu Asp Pro Tyr Leu Pro Gly Asn Pro Arg Glu Leu Ile Ala Tyr Ser
    450                 455                 460

Gln Tyr Pro Arg Pro Ser Asp Ile Pro Gln Trp Asn Ser Asp Lys Pro
465                 470                 475                 480

Ser Leu Lys Asp Ile Lys Ile Met Gly Tyr Ser Ile Arg Thr Ile Asp
                485                 490                 495

Tyr Arg Tyr Thr Val Trp Val Gly Phe Asn Pro Asp Glu Phe Leu Ala
            500                 505                 510

Asn Phe Ser Asp Ile His Ala Gly Glu Leu Tyr Phe Val Asp Ser Asp

515                 520                 525

Pro Leu Gln Asp His Asn Met Tyr Asn Asp Ser Gln Gly Gly Asp Leu
    530                 535                 540

Phe Gln Leu Leu Met Pro Gly Gly Gly Gly Ser Gly Gly Gly Gly Ser
545                 550                 555                 560

Gly Gly Gly Gly Ser Pro Pro Cys Pro Ala Pro Glu Phe Leu Gly Gly
                565                 570                 575

Pro Ser Val Phe Leu Phe Pro Pro Lys Pro Lys Asp Thr Leu Met Ile
            580                 585                 590

Ser Arg Thr Pro Glu Val Thr Cys Val Val Val Asp Val Ser Gln Glu
        595                 600                 605

Asp Pro Glu Val Gln Phe Asn Trp Tyr Val Asp Gly Val Glu Val His
    610                 615                 620

Asn Ala Lys Thr Lys Pro Arg Glu Glu Gln Phe Gln Ser Thr Tyr Arg
625                 630                 635                 640

Val Val Ser Val Leu Thr Val Leu His Gln Asp Trp Leu Asn Gly Lys
                645                 650                 655

Glu Tyr Lys Cys Lys Val Ser Asn Lys Gly Leu Pro Ser Ser Ile Glu
            660                 665                 670

Lys Thr Ile Ser Lys Ala Lys Gly Gln Pro Arg Glu Pro Gln Val Tyr
        675                 680                 685

Thr Leu Pro Pro Ser Gln Glu Glu Met Thr Lys Asn Gln Val Ser Leu
    690                 695                 700

Thr Cys Leu Val Lys Gly Phe Tyr Pro Ser Asp Ile Ala Val Glu Trp
705                 710                 715                 720

Glu Ser Asn Gly Gln Pro Glu Asn Asn Tyr Lys Thr Thr Pro Pro Val
                725                 730                 735

Leu Asp Ser Asp Gly Ser Phe Phe Leu Tyr Ser Arg Leu Thr Val Asp
            740                 745                 750

Lys Ser Arg Trp Gln Glu Gly Asn Val Phe Ser Cys Ser Val Met His
        755                 760                 765

Glu Ala Leu His Asn His Tyr Thr Gln Lys Ser Leu Ser Leu Ser Leu
    770                 775                 780

Gly Lys
785


<210>    24
<211>    2310
<212>    DNA
<213>    Artificial Sequence

<220>
<223>    ARSB-Fc

<400>    24
atgggtccgc gcggcgcggc gagcttgccc cgaggccccg gtcctcggcg gctgcttctc      60

cccgtcgtcc tcccgctgct gctgctgctg ttgttggcgc cgccgggctc gggcgccggg     120

gccagccggc cgccccacct ggtcttcttg ctggcagacg acctaggctg gaacgacgtc     180

ggcttccacg gctcccgcat ccgcacgccg cacctggacg cgctggcggc cggcggggtg     240

ctcctggaca actactacac gcagccgctg tgcacgccgt cgcggagcca gctgctcact     300

ggccgctacc agatccgtac aggtttacag caccaaataa tctggccctg tcagcccagc     360

tgtgttcctc tggatgaaaa actcctgccc cagctcctaa aagaagcagg ttatactacc     420

catatggtcg gaaaatggca cctgggaatg taccggaaag aatgccttcc aacccgccga     480

ggatttgata cctactttgg atatctcctg ggtagtgaag attattattc ccatgaacgc     540

tgtacattaa ttgacgctct gaatgtcaca cgatgtgctc ttgattttcg agatggcgaa     600

gaagttgcaa caggatataa aaatatgtat tcaacaaaca tattcaccaa aagggctata     660

gccctcataa ctaaccatcc accagagaag cctctgtttc tctaccttgc tctccagtct     720

gtgcatgagc cccttcaggt ccctgaggaa tacttgaagc catatgactt tatccaagac     780

aagaacaggc atcactatgc aggaatggtg tcccttatgg atgaagcagt aggaaatgtc     840

actgcagctt taaaaagcag tgggctctgg aacaacacgg tgttcatctt ttctacagat     900

aacggagggc agactttggc agggggtaat aactggcccc ttcgaggaag aaaatggagc     960

ctgtgggaag gaggcgtccg aggggtgggc tttgtggcaa gccccttgct gaagcagaag    1020

ggcgtgaaga accgggagct catccacatc tctgactggc tgccaacact cgtgaagctg    1080

gccaggggac acaccaatgg cacaaagcct ctggatggct tcgacgtgtg gaaaaccatc    1140

agtgaaggaa gcccatcccc cagaattgag ctactgcata atattgaccc gaacttcgtg    1200

gactcttcac cgtgtcccag gaacagcatg gctccagcaa aggatgactc ttctcttcca    1260

gaatattcag cctttaacac atctgtccat gctgcaatta gacatggaaa ttggaaactc    1320

ctcacgggct acccaggctg tggttactgg ttccctccac cgtctcaata caatgtttct    1380

gagataccct catcagaccc accaaccaag accctctggc tctttgatat tgatcgggac    1440

cctgaagaaa gacatgacct gtccagagaa tatcctcaca tcgtcacaaa gctcctgtcc    1500

cgcctacagt tctaccataa acactcagtc cccgtgtact tccctgcaca ggacccccgc    1560

tgtgatccca aggccactgg ggtgtggggc ccttggatgg gtggaggcgg ttcaggcgga    1620

ggtggctctg gcggtggcgg atcgccatca tgcccagcac ctgagttcct ggggggacca    1680

tcagtcttcc tgttcccccc aaaacccaag gacaccctca tgatctcccg gacccctgag    1740

gtcacatgcg tggtggtgga cgtgagccag gaagaccctg aggtccagtt caactggtac    1800

gtggacggcg tggaggtgca taatgccaag acaaagccgc gggaggagca gttcaacagc    1860

acgtaccgtg tggtcagcgt cctcaccgtc ctgcaccagg actggctgaa tggcaaggag 1920

tacaagtgca aggtctccaa caaaggcctc ccatcctcca tcgagaaaac catctccaaa 1980

gccaaagggc agccccgaga accacaggtg tacaccctgc ccccatccca ggaggagatg 2040

accaagaacc aggtcagcct gacctgcctg gtcaaaggct tctatcccag cgacatcgcc 2100

gtggagtggg agagcaatgg gcagccggag aacaactaca agaccacgcc tcccgtgctg 2160

gactccgacg gctccttctt cctctacagc aggctaaccg tggacaagag caggtggcag 2220

gaggggaacg tcttctcatg ctccgtgatg catgaggctc tgcacaacca ctacacgcag 2280

aagagcctct ccctgtctct gggtaaatga 2310

<210> 25
<211> 769
<212> PRT
<213> Artificial Sequence

<220>
<223> ARSB-Fc

<400> 25
   Met Gly Pro Arg Gly Ala Ala Ser Leu Pro Arg Gly Pro Gly Pro Arg
   1 5 10 15
   Arg Leu Leu Leu Pro Val Val Leu Pro Leu Leu Leu Leu Leu Leu Leu
   20 25 30
   Ala Pro Pro Gly Ser Gly Ala Gly Ala Ser Arg Pro Pro His Leu Val
   35 40 45
   Phe Leu Leu Ala Asp Asp Leu Gly Trp Asn Asp Val Gly Phe His Gly
   50 55 60
   Ser Arg Ile Arg Thr Pro His Leu Asp Ala Leu Ala Ala Gly Gly Val
   65 70 75 80
   Leu Leu Asp Asn Tyr Tyr Thr Gln Pro Leu Cys Thr Pro Ser Arg Ser
   85 90 95
   Gln Leu Leu Thr Gly Arg Tyr Gln Ile Arg Thr Gly Leu Gln His Gln
   100 105 110
   Ile Ile Trp Pro Cys Gln Pro Ser Cys Val Pro Leu Asp Glu Lys Leu
   115 120 125
   Leu Pro Gln Leu Leu Lys Glu Ala Gly Tyr Thr Thr His Met Val Gly
   130 135 140
   Lys Trp His Leu Gly Met Tyr Arg Lys Glu Cys Leu Pro Thr Arg Arg
   145 150 155 160
   Gly Phe Asp Thr Tyr Phe Gly Tyr Leu Leu Gly Ser Glu Asp Tyr Tyr
   165 170 175
   Ser His Glu Arg Cys Thr Leu Ile Asp Ala Leu Asn Val Thr Arg Cys
   180 185 190
   Ala Leu Asp Phe Arg Asp Gly Glu Glu Val Ala Thr Gly Tyr Lys Asn
           195                 200                 205
   Met Tyr Ser Thr Asn Ile Phe Thr Lys Arg Ala Ile Ala Leu Ile Thr
       210                 215                 220
   Asn His Pro Pro Glu Lys Pro Leu Phe Leu Tyr Leu Ala Leu Gln Ser
   225                 230                 235                 240
   Val His Glu Pro Leu Gln Val Pro Glu Glu Tyr Leu Lys Pro Tyr Asp
                   245                 250                 255
   Phe Ile Gln Asp Lys Asn Arg His His Tyr Ala Gly Met Val Ser Leu
               260                 265                 270
   Met Asp Glu Ala Val Gly Asn Val Thr Ala Ala Leu Lys Ser Ser Gly
           275                 280                 285
   Leu Trp Asn Asn Thr Val Phe Ile Phe Ser Thr Asp Asn Gly Gly Gln
       290                 295                 300
   Thr Leu Ala Gly Gly Asn Asn Trp Pro Leu Arg Gly Arg Lys Trp Ser
   305                 310                 315                 320
   Leu Trp Glu Gly Gly Val Arg Gly Val Gly Phe Val Ala Ser Pro Leu
                   325                 330                 335
   Leu Lys Gln Lys Gly Val Lys Asn Arg Glu Leu Ile His Ile Ser Asp
               340                 345                 350
   Trp Leu Pro Thr Leu Val Lys Leu Ala Arg Gly His Thr Asn Gly Thr
           355                 360                 365
   Lys Pro Leu Asp Gly Phe Asp Val Trp Lys Thr Ile Ser Glu Gly Ser
       370                 375                 380
   Pro Ser Pro Arg Ile Glu Leu Leu His Asn Ile Asp Pro Asn Phe Val
   385                 390                 395                 400
   Asp Ser Ser Pro Cys Pro Arg Asn Ser Met Ala Pro Ala Lys Asp Asp
                   405                 410                 415
   Ser Ser Leu Pro Glu Tyr Ser Ala Phe Asn Thr Ser Val His Ala Ala
               420                 425                 430
   Ile Arg His Gly Asn Trp Lys Leu Leu Thr Gly Tyr Pro Gly Cys Gly
           435                 440                 445
   Tyr Trp Phe Pro Pro Pro Ser Gln Tyr Asn Val Ser Glu Ile Pro Ser
       450                 455                 460
   Ser Asp Pro Pro Thr Lys Thr Leu Trp Leu Phe Asp Ile Asp Arg Asp
   465                 470                 475                 480
   Pro Glu Glu Arg His Asp Leu Ser Arg Glu Tyr Pro His Ile Val Thr
                   485                 490                 495
   Lys Leu Leu Ser Arg Leu Gln Phe Tyr His Lys His Ser Val Pro Val
               500                 505                 510
   Tyr Phe Pro Ala Gln Asp Pro Arg Cys Asp Pro Lys Ala Thr Gly Val
           515                 520                 525
   Trp Gly Pro Trp Met Gly Gly Gly Gly Ser Gly Gly Gly Gly Ser Gly
       530                 535                 540
   Gly Gly Gly Ser Pro Pro Cys Pro Ala Pro Glu Phe Leu Gly Gly Pro
   545                 550                 555                 560
   Ser Val Phe Leu Phe Pro Pro Lys Pro Lys Asp Thr Leu Met Ile Ser
                   565                 570                 575
   Arg Thr Pro Glu Val Thr Cys Val Val Val Asp Val Ser Gln Glu Asp
               580                 585                 590
   Pro Glu Val Gln Phe Asn Trp Tyr Val Asp Gly Val Glu Val His Asn
           595                 600                 605
   Ala Lys Thr Lys Pro Arg Glu Glu Gln Phe Gln Ser Thr Tyr Arg Val
       610                 615                 620
   Val Ser Val Leu Thr Val Leu His Gln Asp Trp Leu Asn Gly Lys Glu
   625                 630                 635                 640
   Tyr Lys Cys Lys Val Ser Asn Lys Gly Leu Pro Ser Ser Ile Glu Lys
                   645                 650                 655
   Thr Ile Ser Lys Ala Lys Gly Gln Pro Arg Glu Pro Gln Val Tyr Thr
               660                 665                 670
   Leu Pro Pro Ser Gln Glu Glu Met Thr Lys Asn Gln Val Ser Leu Thr
           675                 680                 685
   Cys Leu Val Lys Gly Phe Tyr Pro Ser Asp Ile Ala Val Glu Trp Glu
       690                 695                 700
   Ser Asn Gly Gln Pro Glu Asn Asn Tyr Lys Thr Thr Pro Pro Val Leu
   705                 710                 715                 720
   Asp Ser Asp Gly Ser Phe Phe Leu Tyr Ser Arg Leu Thr Val Asp Lys
                   725                 730                 735
   Ser Arg Trp Gln Glu Gly Asn Val Phe Ser Cys Ser Val Met His Glu
               740                 745                 750
   Ala Leu His Asn His Tyr Thr Gln Lys Ser Leu Ser Leu Ser Leu Gly
           755                 760                 765
   Lys

<210>    26
<211>    12
<212>    PRT
<213>    Homo Sapiens

<400>    26
Glu Ser Lys Tyr Gly Pro Pro Cys Pro Ser Cys Pro
1               5                   10